# EUROPEAN PATENT SPECIFICATION

(11) **EP 2 764 018 B1**
(45) Date of publication and mention of the grant of the patent: **11.10.2017**
(21) Application number: 12791872.0
(22) Date of filing: 29.08.2012
(51) Int. Cl.: C07K 14/435, B82B 1/00

(54) **SELF-ASSEMBLING POLYPEPTIDE POLYHEDRA**
SELBSTANORDNENDES POLYPEPTID-POLYEDER
POLYÈDRES POLYPEPTIDIQUES AUTO-ASSEMBLÉS

(30) Priority: 07.10.2011 SI 201100402
(43) Date of publication of application: 13.08.2014
(73) Proprietor: Kemijski Institut, 1000 Ljubljana (SI); En-Fist Center Odlicnosti (Zavod), 1000 Ljubljana (SI)
(72) Inventor: JERALA, Roman, SI-1000 Ljubljana (SI); GRADISAR, Helena, SI- 1000 Ljubljana (SI); BOZIC, Sabina, SI-6333 Secovlje (SI); DOLES, Tibor, SI-5263 Dobravlje (SI)
(74) Representative: Grünecker Patent- und Rechtsanwälte PartG mbB
(86) International application number: PCT/SI2012/000052
(87) International publication number: WO 2013/052015

(56) References cited:
- WO-A1-2011/046521
- ROTHEMUND P W K: "Folding DNA to create nanoscale shapes and patterns", NATURE: INTERNATIONAL WEEKLY JOURNAL OF SCIENCE, NATURE PUBLISHING GROUP, UNITED KINGDOM, vol. 440, 16 March 2006 (2006-03-16), pages 297-302, XP002598319, ISSN: 0028-0836, DOI: 10.1038/NATURE04586 cited in the application

### SEQUENCE LISTING

<110> Kemijski inštitut, Ljubljana, Slovenia and EN-FIST Center odličnosti, Ljubljana, Slovenia
<120> Self-assembling polypeptide polyhedra
<130> 301-P18PC/12
<140> -
   <141> 2012-29-08
<150> SI P-201100402
   <151> 2011-10-07
<160> 22
<170> PatentIn version 3.4
<210> 1
   <211> 135
   <212> DNA
   <213> synthetic sequence
<220>
   <221> CDS
   <222> (1)..(135)
   <223> APH
<400> 1
   atg aaa cag ctg gaa aaa gag ctg aaa cag tta gaa aaa gaa ctg caa       48
   Met Lys Gln Leu Glu Lys Glu Leu Lys Gln Leu Glu Lys Glu Leu Gln
   1               5                   10                  15
   gca att gaa aaa cag ctg gca cag ctg caa tgg aaa gca cag gca cgt       96
   Ala Ile Glu Lys Gln Leu Ala Gln Leu Gln Trp Lys Ala Gln Ala Arg
               20                  25                  30
   aaa aaa aaa ctg gcc cag ctg aaa aaa aaa ctt cag gcc                   135
   Lys Lys Lys Leu Ala Gln Leu Lys Lys Lys Leu Gln Ala
           35                  40                  45
<210> 2
   <211> 45
   <212> PRT
   <213> synthetic sequence
<220>
   <223> APH, coiled-coil-forming segment
<400> 2
   Met Lys Gln Leu Glu Lys Glu Leu Lys Gln Leu Glu Lys Glu Leu Gln
   1               5                   10                  15
   Ala Ile Glu Lys Gln Leu Ala Gln Leu Gln Trp Lys Ala Gln Ala Arg
               20                  25                  30
   Lys Lys Lys Leu Ala Gln Leu Lys Lys Lys Leu Gln Ala
           35                  40                  45
<210> 3
   <211> 111
   <212> DNA
   <213> synthetic protein
<220>
   <221> CDS
   <222> (1)..(111)
   <223> BCR
<400> 3
   atg gat att gaa cag gaa ctg gaa cgc gca aaa gca agc att cgt cgt        48
   Met Asp Ile Glu Gln Glu Leu Glu Arg Ala Lys Ala Ser Ile Arg Arg
   1               5                   10                  15
   ctg gaa cag gaa gtt aat caa gaa cgt agc cgt atg gca tat ctg caa        96
   Leu Glu Gln Glu Val Asn Gln Glu Arg Ser Arg Met Ala Tyr Leu Gln
               20                  25                  30
   acc ctg ctg gca aaa                                                   111
   Thr Leu Leu Ala Lys
           35
<210> 4
   <211> 37
   <212> PRT
   <213> synthetic protein
<220>
   <223> BCR, coiled-coil-forming segment
<400> 4
   Met Asp Ile Glu Gln Glu Leu Glu Arg Ala Lys Ala Ser Ile Arg Arg
   1               5                   10                  15
   Leu Glu Gln Glu Val Asn Gln Glu Arg Ser Arg Met Ala Tyr Leu Gln
               20                  25                  30
   Thr Leu Leu Ala Lys
           35
<210> 5
   <211> 84
   <212> DNA
   <213> Synthetic sequence
<220>
   <221> CDS
   <222> (1)..(84)
   <223> GCNshort
<400> 5
   cag tta gaa gac aaa gtt gaa gaa ctg ctg agc aag aac tac cat ctg        48
   Gln Leu Glu Asp Lys Val Glu Glu Leu Leu Ser Lys Asn Tyr His Leu
   1               5                   10                  15
   gaa aat gaa gtt gcc cgt ctg aaa aaa ctg gtt ggt                        84
   Glu Asn Glu Val Ala Arg Leu Lys Lys Leu Val Gly
                                   20                  25
<210> 6
   <211> 33
   <212> PRT
   <213> Synthetic
<220>
   <223> GCN short, coiled-coil-forming segment
<400> 6
<210> 7
   <211> 99
   <212> DNA
   <213> Synthetic
<220>
   <221> CDS
   <222> (1)..(99)
   <223> P3
<400> 7
   tcc ccg gaa gat gag atc cag caa ctg gaa gaa gaa atc gct cag ctg      48
   Ser Pro Glu Asp Glu Ile Gln Gln Leu Glu Glu Glu Ile Ala Gln Leu
   1               5                   10                  15
   gaa cag aaa aac gca gcg ctg aaa gag aaa aac cag gcg ctg aaa tac      96
   Glu Gln Lys Asn Ala Ala Leu Lys Glu Lys Asn Gln Ala Leu Lys Tyr
               20                  25                  30
   ggt                                                                  99
   Gly
<210> 8
   <211> 33
   <212> PRT
   <213> Synthetic
<220>
   <223> P3, coiled-coil-forming segment
<400> 8
   Ser Pro Glu Asp Glu Ile Gln Gln Leu Glu Glu Glu Ile Ala Gln Leu
   1               5                   10                  15
   Glu Gln Lys Asn Ala Ala Leu Lys Glu Lys Asn Gln Ala Leu Lys Tyr
               20                  25                  30
   Gly
<210> 9
   <211> 99
   <212> DNA
   <213> Synthetic
<220>
   <221> CDS
   <222> (1)..(99)
   <223> P4
<400> 9
   agc ccg gaa gat aaa att gct cag ctg aaa caa aaa atc caa gcg ctg       48
   Ser Pro Glu Asp Lys Ile Ala Gln Leu Lys Gln Lys Ile Gln Ala Leu
   1               5                   10                  15
   aaa cag gaa aac cag cag ctg gaa gag gaa aac gcc gca ctg gaa tat       96
   Lys Gln Glu Asn Gln Gln Leu Glu Glu Glu Asn Ala Ala Leu Glu Tyr
               20                  25                  30
   ggt                                                                   99
   Gly
<210> 10
   <211> 33
   <212> PRT
   <213> Synthetic
<220>
   <223> P4, coiled-coil-forming segment
<400> 10
   Ser Pro Glu Asp Lys Ile Ala Gln Leu Lys Gln Lys Ile Gln Ala Leu
   1               5                   10                  15
   Lys Gln Glu Asn Gln Gln Leu Glu Glu Glu Asn Ala Ala Leu Glu Tyr
               20                  25                  30
   Gly
<210> 11
   <211> 99
   <212> DNA
   <213> Synthetic
<220>
   <221> CDS
   <222> (1)..(99)
   <223> P5
<400> 11
   tct cct gaa gac gaa aac gca gct ctg gaa gag aaa att gca caa ctg       48
   Ser Pro Glu Asp Glu Asn Ala Ala Leu Glu Glu Lys Ile Ala Gln Leu
   1               5                   10                  15
   aaa caa aag aac gcg gca ctg aaa gaa gaa att caa gca ctg gaa tat       96
   Lys Gln Lys Asn Ala Ala Leu Lys Glu Glu Ile Gln Ala Leu Glu Tyr
               20                  25                  30
   ggc                                                                   99
   Gly
<210> 12
   <211> 33
   <212> PRT
   <213> Synthetic
<220>
   <223> P5, coiled-coil-forming segment
<400> 12
   Ser Pro Glu Asp Glu Asn Ala Ala Leu Glu Glu Lys Ile Ala Gln Leu
   1               5                   10                  15
   Lys Gln Lys Asn Ala Ala Leu Lys Glu Glu Ile Gln Ala Leu Glu Tyr
               20                  25                  30
   Gly
<210> 13
   <211> 99
   <212> DNA
   <213> Synthetic
<220>
   <221> CDS
   <222> (1)..(99)
   <223> P6
<400> 13
   agc ccg gaa gat aaa aac gcc gct ctg aaa gag gaa atc cag gcg ctg       48
   Ser Pro Glu Asp Lys Asn Ala Ala Leu Lys Glu Glu Ile Gln Ala Leu
   1               5                   10                  15
   gaa gaa gaa aac cag gct ctg gaa gag aaa atc gca cag ctg aaa tat       96
   Glu Glu Glu Asn Gln Ala Leu Glu Glu Lys Ile Ala Gln Leu Lys Tyr
               20                  25                  30
   ggt                                                                   99
   Gly
<210> 14
   <211> 33
   <212> PRT
   <213> Synthetic
<220>
   <223> P6, coiled-coil-forming segment
<400> 14
   Ser Pro Glu Asp Lys Asn Ala Ala Leu Lys Glu Glu Ile Gln Ala Leu
   1               5                   10                  15
   Glu Glu Glu Asn Gln Ala Leu Glu Glu Lys Ile Ala Gln Leu Lys Tyr
               20                  25                  30
   Gly
<210> 15
   <211> 99
   <212> DNA
   <213> Synthetic
<220>
   <221> CDS
   <222> (1)..(99)
   <223> P7
<400> 15
   tcc ccg gag gat gag atc cag gcg ctg gaa gaa aag aac gcc cag ctg       48
   Ser Pro Glu Asp Glu Ile Gln Ala Leu Glu Glu Lys Asn Ala Gln Leu
   1               5                   10                  15
   aag cag gaa att gcg gca ctg gaa gag aag aac cag gcc ctg aag tac       96
   Lys Gln Glu Ile Ala Ala Leu Glu Glu Lys Asn Gln Ala Leu Lys Tyr
               20                  25                  30
   ggt                                                                   99
   Gly
<210> 16
   <211> 33
   <212> PRT
   <213> Synthetic
<220>
   <223> P7, coiled-coil-forming segment
<400> 16
   Ser Pro Glu Asp Glu Ile Gln Ala Leu Glu Glu Lys Asn Ala Gln Leu
   1               5                   10                  15
   Lys Gln Glu Ile Ala Ala Leu Glu Glu Lys Asn Gln Ala Leu Lys Tyr
               20                  25                  30
   Gly
<210> 17
   <211> 99
   <212> DNA
   <213> Synthetic
<220>
   <221> CDS
   <222> (1)..(99)
   <223> P8
<400> 17
   tcc ccg gaa gac aaa atc gct cag ctg aaa gaa gaa aac cag cag ctg       48
   Ser Pro Glu Asp Lys Ile Ala Gln Leu Lys Glu Glu Asn Gln Gln Leu
   1               5                   10                  15
   gaa caa aag att cag gcc ctg aag gag gaa aac gca gct ctg gaa tac       96
   Glu Gln Lys Ile Gln Ala Leu Lys Glu Glu Asn Ala Ala Leu Glu Tyr
               20                  25                  30
   ggc                                                                   99
   Gly
<210> 18
   <211> 33
   <212> PRT
   <213> Synthetic
<220>
   <223> P8, coiled-coil-forming segment
<400> 18
   Ser Pro Glu Asp Lys Ile Ala Gln Leu Lys Glu Glu Asn Gln Gln Leu
   1               5                   10                  15
   Glu Gln Lys Ile Gln Ala Leu Lys Glu Glu Asn Ala Ala Leu Glu Tyr
               20                  25                  30
   Gly
<210> 19
   <211> 1380
   <212> DNA
   <213> Synthetic sequence
<220>
   <221> CDS
   <222> (1)..(1380)
   <223> TET12
<400> 19
   atg aaa cag ctg gaa aaa gaa ctg aaa cag tta gag aaa gag ctg caa        48
   Met Lys Gln Leu Glu Lys Glu Leu Lys Gln Leu Glu Lys Glu Leu Gln
   1               5                   10                  15
   gcc att gaa aaa cag ctg gca cag ctg caa tgg aaa gca cag gca cgt        96
   Ala Ile Glu Lys Gln Leu Ala Gln Leu Gln Trp Lys Ala Gln Ala Arg
               20                  25                  30
   aaa aag aaa ctg gcc cag ctg aaa aaa aag ctg caa gca agc ggt ccg       144
   Lys Lys Lys Leu Ala Gln Leu Lys Lys Lys Leu Gln Ala Ser Gly Pro
           35                  40                  45
   ggt tca ccg gaa gat aaa att gca cag ctg aaa gaa aaa aat gca gcc       192
   Gly Ser Pro Glu Asp Lys Ile Ala Gln Leu Lys Glu Lys Asn Ala Ala
       50                  55                  60
   ctg aaa gag aaa aac cag caa ctg aaa gaa aag att cag gcg ctg aaa       240
   Leu Lys Glu Lys Asn Gln Gln Leu Lys Glu Lys Ile Gln Ala Leu Lys
   65                  70                  75                  80
   tat ggt agt ggt ccg ggt gat att gaa caa gaa ctg gaa cgt gca aaa       288
   Tyr Gly Ser Gly Pro Gly Asp Ile Glu Gln Glu Leu Glu Arg Ala Lys
                   85                  90                  95
   gca agc att cgt cgt ctg gaa caa gaa gtt aat caa gaa cgt agc cgc       336
   Ala Ser Ile Arg Arg Leu Glu Gln Glu Val Asn Gln Glu Arg Ser Arg
               100                 105                 110
   atg gca tat ctg caa acc ctg ctg gca aaa tca ggt ccg ggt cag tta       384
   Met Ala Tyr Leu Gln Thr Leu Leu Ala Lys Ser Gly Pro Gly Gln Leu
           115                 120                 125
   gaa gac aaa gtt gaa gaa ctg ctg agc aag aac tac cat ctg gaa aat        432
   Glu Asp Lys Val Glu Glu Leu Leu Ser Lys Asn Tyr His Leu Glu Asn
       130                 135                 140
   gaa gtt gcc cgt ctg aaa aaa ctg gtt ggt tct ggt ccg ggt atg aag        480
   Glu Val Ala Arg Leu Lys Lys Leu Val Gly Ser Gly Pro Gly Met Lys
   145                 150                 155                 160
   caa tta gaa aaa gag tta aaa caa ctg gaa aaa gag tta cag gcc atc        528
   Gln Leu Glu Lys Glu Leu Lys Gln Leu Glu Lys Glu Leu Gln Ala Ile
                   165                 170                 175
   gag aaa cag tta gca cag tta cag tgg aaa gcc cag gct cgc aaa aaa        576
   Glu Lys Gln Leu Ala Gln Leu Gln Trp Lys Ala Gln Ala Arg Lys Lys
               180                 185                 190
   aag tta gcc caa ctg aag aag aaa tta cag gca tca ggt cct ggt agc        624
   Lys Leu Ala Gln Leu Lys Lys Lys Leu Gln Ala Ser Gly Pro Gly Ser
           195                 200                 205
   cct gaa gat gaa atc cag gca ctg gaa gaa aaa aac gcg cag ctg aaa        672
   Pro Glu Asp Glu Ile Gln Ala Leu Glu Glu Lys Asn Ala Gln Leu Lys
       210                 215                 220
   caa gaa att gcc gca tta gag gaa aaa aac cag gca ctg aaa tac ggt        720
   Gln Glu Ile Ala Ala Leu Glu Glu Lys Asn Gln Ala Leu Lys Tyr Gly
   225                 230                 235                 240
   tct ggt cct ggt caa ctg gaa gat aag gtg gaa gaa tta ctg tcg aaa        768
   Ser Gly Pro Gly Gln Leu Glu Asp Lys Val Glu Glu Leu Leu Ser Lys
                   245                 250                 255
   aac tat cat tta gaa aac gaa gtg gca cgc ctg aaa aag tta gtt ggt        816
   Asn Tyr His Leu Glu Asn Glu Val Ala Arg Leu Lys Lys Leu Val Gly
               260                 265                 270
   agc ggt cca ggt agt cca gaa gat aaa atc gcc cag tta aaa cag aaa        864
   Ser Gly Pro Gly Ser Pro Glu Asp Lys Ile Ala Gln Leu Lys Gln Lys
           275                 280                 285
   atc cag gcc ctg aaa caa gag aat cag cag ctg gaa gag gaa aat gca        912
   Ile Gln Ala Leu Lys Gln Glu Asn Gln Gln Leu Glu Glu Glu Asn Ala
       290                 295                 300
   gca ctg gaa tat ggc agc ggt cct ggc tct ccc gaa gat gaa aac gct        960
   Ala Leu Glu Tyr Gly Ser Gly Pro Gly Ser Pro Glu Asp Glu Asn Ala
   305                 310                 315                 320
   gcc tta gaa gag aaa att gcg caa ctg aag cag aag aat gcc gca ctg       1008
   Ala Leu Glu Glu Lys Ile Ala Gln Leu Lys Gln Lys Asn Ala Ala Leu
                   325                 330                 335
   aaa gag gaa att cag gcc ctg gaa tac ggc tca ggt cca ggt tct cct       1056
   Lys Glu Glu Ile Gln Ala Leu Glu Tyr Gly Ser Gly Pro Gly Ser Pro
               340                 345                 350
   gag gac aaa atc gca caa tta aaa gaa gaa aac caa cag ctg gaa cag       1104
   Glu Asp Lys Ile Ala Gln Leu Lys Glu Glu Asn Gln Gln Leu Glu Gln
           355                 360                 365
   aaa att caa gcc tta aaa gaa gag aac gct gcc ctg gaa tat ggt tct      1152
   Lys Ile Gln Ala Leu Lys Glu Glu Asn Ala Ala Leu Glu Tyr Gly Ser
       370                 375                 380
   ggc cct ggc gat atc gaa caa gaa tta gaa aga gcc aaa gcc agt atc      1200
   Gly Pro Gly Asp Ile Glu Gln Glu Leu Glu Arg Ala Lys Ala Ser Ile
   385                 390                 395                 400
   cgt cgt tta gaa caa gag gta aac caa gag cgt tca cgt atg gca tat      1248
   Arg Arg Leu Glu Gln Glu Val Asn Gln Glu Arg Ser Arg Met Ala Tyr
                   405                 410                 415
   tta cag aca ctg ctg gcc aaa agt ggt cct ggc tcc cca gag gat aaa      1296
   Leu Gln Thr Leu Leu Ala Lys Ser Gly Pro Gly Ser Pro Glu Asp Lys
               420                 425                 430
   aat gcc gct tta aaa gag gaa atc caa gcc tta gaa gag gaa aac cag      1344
   Asn Ala Ala Leu Lys Glu Glu Ile Gln Ala Leu Glu Glu Glu Asn Gln
           435                 440                 445
   gcg tta gag gaa aag att gct cag ctg aag tat ggt                      1380
   Ala Leu Glu Glu Lys Ile Ala Gln Leu Lys Tyr Gly
       450                 455                 460
<210> 20
   <211> 460
   <212> PRT
   <213> Synthetic sequence
<220>
   <223> TET12, tetrahedron-forming polypeptide sequence
<400> 20
   Met Lys Gln Leu Glu Lys Glu Leu Lys Gln Leu Glu Lys Glu Leu Gln
   1               5                   10                  15
   Ala Ile Glu Lys Gln Leu Ala Gln Leu Gln Trp Lys Ala Gln Ala Arg
               20                  25                  30
   Lys Lys Lys Leu Ala Gln Leu Lys Lys Lys Leu Gln Ala Ser Gly Pro
           35                  40                  45
   Gly Ser Pro Glu Asp Lys Ile Ala Gln Leu Lys Glu Lys Asn Ala Ala
       50                  55                  60
   Leu Lys Glu Lys Asn Gln Gln Leu Lys Glu Lys Ile Gln Ala Leu Lys
   65                  70                  75                  80
   Tyr Gly Ser Gly Pro Gly Asp Ile Glu Gln Glu Leu Glu Arg Ala Lys
                   85                  90                  95
   Ala Ser Ile Arg Arg Leu Glu Gln Glu Val Asn Gln Glu Arg Ser Arg
               100                 105                 110
   Met Ala Tyr Leu Gln Thr Leu Leu Ala Lys Ser Gly Pro Gly Gln Leu
           115                 120                 125
   Glu Asp Lys Val Glu Glu Leu Leu Ser Lys Asn Tyr His Leu Glu Asn
       130                 135                 140
   Glu Val Ala Arg Leu Lys Lys Leu Val Gly Ser Gly Pro Gly Met Lys
   145                 150                 155                 160
   Gln Leu Glu Lys Glu Leu Lys Gln Leu Glu Lys Glu Leu Gln Ala Ile
                   165                 170                 175
   Glu Lys Gln Leu Ala Gln Leu Gln Trp Lys Ala Gln Ala Arg Lys Lys
               180                 185                 190
   Lys Leu Ala Gln Leu Lys Lys Lys Leu Gln Ala Ser Gly Pro Gly Ser
           195                 200                 205
   Pro Glu Asp Glu Ile Gln Ala Leu Glu Glu Lys Asn Ala Gln Leu Lys
       210                 215                 220
   Gln Glu Ile Ala Ala Leu Glu Glu Lys Asn Gln Ala Leu Lys Tyr Gly
   225                 230                 235                 240
   Ser Gly Pro Gly Gln Leu Glu Asp Lys Val Glu Glu Leu Leu Ser Lys
                   245                 250                 255
   Asn Tyr His Leu Glu Asn Glu Val Ala Arg Leu Lys Lys Leu Val Gly
               260                 265                 270
   Ser Gly Pro Gly Ser Pro Glu Asp Lys Ile Ala Gln Leu Lys Gln Lys
           275                 280                 285
   Ile Gln Ala Leu Lys Gln Glu Asn Gln Gln Leu Glu Glu Glu Asn Ala
       290                 295                 300
   Ala Leu Glu Tyr Gly Ser Gly Pro Gly Ser Pro Glu Asp Glu Asn Ala
   305                 310                 315                 320
   Ala Leu Glu Glu Lys Ile Ala Gln Leu Lys Gln Lys Asn Ala Ala Leu
                   325                 330                 335
   Lys Glu Glu Ile Gln Ala Leu Glu Tyr Gly Ser Gly Pro Gly Ser Pro
               340                 345                 350
   Glu Asp Lys Ile Ala Gln Leu Lys Glu Glu Asn Gln Gln Leu Glu Gln
           355                 360                 365
   Lys Ile Gln Ala Leu Lys Glu Glu Asn Ala Ala Leu Glu Tyr Gly Ser
       370                 375                 380
   Gly Pro Gly Asp Ile Glu Gln Glu Leu Glu Arg Ala Lys Ala Ser Ile
   385                 390                 395                 400
   Arg Arg Leu Glu Gln Glu Val Asn Gln Glu Arg Ser Arg Met Ala Tyr
                   405                 410                 415
   Leu Gln Thr Leu Leu Ala Lys Ser Gly Pro Gly Ser Pro Glu Asp Lys
               420                 425                 430
   Asn Ala Ala Leu Lys Glu Glu Ile Gln Ala Leu Glu Glu Glu Asn Gln
           435                 440                 445
   Ala Leu Glu Glu Lys Ile Ala Gln Leu Lys Tyr Gly
       450                 455                 460
<210> 21
   <211> 2112
   <212> DNA
   <213> Synthetic sequence
<220>
   <221> CDS
   <222> (1)..(2112)
   <223> TET12 split YFP
<400> 21
   gat aaa cag aaa aat ggc atc aaa gtg aac ttc aaa atc cgc cac aac        48
   Asp Lys Gln Lys Asn Gly Ile Lys Val Asn Phe Lys Ile Arg His Asn
   1               5                   10                  15
   atc gag gat ggt agt gta caa ctg gcc gat cac tat caa caa aac acc        96
   Ile Glu Asp Gly Ser Val Gln Leu Ala Asp His Tyr Gln Gln Asn Thr
               20                  25                  30
   ccg att ggt gac ggg ccg gtt ctg ctg cct gat aac cat tat ctg tca       144
   Pro Ile Gly Asp Gly Pro Val Leu Leu Pro Asp Asn His Tyr Leu Ser
           35                  40                  45
   tat caa tct gcc ctg tcc aaa gat ccg aac gag aaa cgt gac cac atg       192
   Tyr Gln Ser Ala Leu Ser Lys Asp Pro Asn Glu Lys Arg Asp His Met
       50                  55                  60
   gta ctg ctg gaa ttt gtg act gct gcc ggt att acc ctg ggt atg gac      240
   Val Leu Leu Glu Phe Val Thr Ala Ala Gly Ile Thr Leu Gly Met Asp
   65                  70                  75                  80
   gaa ctg tat aaa atg aaa cag ctg gaa aaa gaa ctg aaa cag tta gag      288
   Glu Leu Tyr Lys Met Lys Gln Leu Glu Lys Glu Leu Lys Gln Leu Glu
                   85                  90                  95
   aaa gag ctg caa gcc att gaa aaa cag ctg gca cag ctg caa tgg aaa      336
   Lys Glu Leu Gln Ala Ile Glu Lys Gln Leu Ala Gln Leu Gln Trp Lys
               100                 105                 110
   gca cag gca cgt aaa aag aaa ctg gcc cag ctg aaa aaa aag ctg caa      384
   Ala Gln Ala Arg Lys Lys Lys Leu Ala Gln Leu Lys Lys Lys Leu Gln
           115                 120                 125
   gca agc ggt ccg ggt tca ccg gaa gat aaa att gca cag ctg aaa gaa      432
   Ala Ser Gly Pro Gly Ser Pro Glu Asp Lys Ile Ala Gln Leu Lys Glu
       130                 135                 140
   aaa aat gca gcc ctg aaa gag aaa aac cag caa ctg aaa gaa aag att      480
   Lys Asn Ala Ala Leu Lys Glu Lys Asn Gln Gln Leu Lys Glu Lys Ile
   145                 150                 155                 160
   cag gcg ctg aaa tat ggt agt ggt ccg ggt gat att gaa caa gaa ctg      528
   Gln Ala Leu Lys Tyr Gly Ser Gly Pro Gly Asp Ile Glu Gln Glu Leu
                   165                 170                 175
   gaa cgt gca aaa gca agc att cgt cgt ctg gaa caa gaa gtt aat caa      576
   Glu Arg Ala Lys Ala Ser Ile Arg Arg Leu Glu Gln Glu Val Asn Gln
               180                 185                 190
   gaa cgt agc cgc atg gca tat ctg caa acc ctg ctg gca aaa tca ggt      624
   Glu Arg Ser Arg Met Ala Tyr Leu Gln Thr Leu Leu Ala Lys Ser Gly
           195                 200                 205
   ccg ggt cag tta gaa gac aaa gtt gaa gaa ctg ctg agc aag aac tac      672
   Pro Gly Gln Leu Glu Asp Lys Val Glu Glu Leu Leu Ser Lys Asn Tyr
       210                 215                 220
   cat ctg gaa aat gaa gtt gcc cgt ctg aaa aaa ctg gtt ggt tct ggt      720
   His Leu Glu Asn Glu Val Ala Arg Leu Lys Lys Leu Val Gly Ser Gly
   225                 230                 235                 240
   ccg ggt atg aag caa tta gaa aaa gag tta aaa caa ctg gaa aaa gag      768
   Pro Gly Met Lys Gln Leu Glu Lys Glu Leu Lys Gln Leu Glu Lys Glu
                   245                 250                 255
   tta cag gcc atc gag aaa cag tta gca cag tta cag tgg aaa gcc cag      816
   Leu Gln Ala Ile Glu Lys Gln Leu Ala Gln Leu Gln Trp Lys Ala Gln
               260                 265                 270
   gct cgc aaa aaa aag tta gcc caa ctg aag aag aaa tta cag gca tca      864
   Ala Arg Lys Lys Lys Leu Ala Gln Leu Lys Lys Lys Leu Gln Ala Ser
           275                 280                 285
   ggt cct ggt agc cct gaa gat gaa atc cag gca ctg gaa gaa aaa aac      912
   Gly Pro Gly Ser Pro Glu Asp Glu Ile Gln Ala Leu Glu Glu Lys Asn
       290                 295                 300
   gcg cag ctg aaa caa gaa att gcc gca tta gag gaa aaa aac cag gca      960
   Ala Gln Leu Lys Gln Glu Ile Ala Ala Leu Glu Glu Lys Asn Gln Ala
   305                 310                 315                 320
   ctg aaa tac ggt tct ggt cct ggt caa ctg gaa gat aag gtg gaa gaa     1008
   Leu Lys Tyr Gly Ser Gly Pro Gly Gln Leu Glu Asp Lys Val Glu Glu
                   325                 330                 335
   tta ctg tcg aaa aac tat cat tta gaa aac gaa gtg gca cgc ctg aaa     1056
   Leu Leu Ser Lys Asn Tyr His Leu Glu Asn Glu Val Ala Arg Leu Lys
               340                 345                 350
   aag tta gtt ggt agc ggt cca ggt agt cca gaa gat aaa atc gcc cag     1104
   Lys Leu Val Gly Ser Gly Pro Gly Ser Pro Glu Asp Lys Ile Ala Gln
           355                 360                 365
   tta aaa cag aaa atc cag gcc ctg aaa caa gag aat cag cag ctg gaa     1152
   Leu Lys Gln Lys Ile Gln Ala Leu Lys Gln Glu Asn Gln Gln Leu Glu
       370                 375                 380
   gag gaa aat gca gca ctg gaa tat ggc agc ggt cct ggc tct ccc gaa     1200
   Glu Glu Asn Ala Ala Leu Glu Tyr Gly Ser Gly Pro Gly Ser Pro Glu
   385                 390                 395                 400
   gat gaa aac gct gcc tta gaa gag aaa att gcg caa ctg aag cag aag     1248
   Asp Glu Asn Ala Ala Leu Glu Glu Lys Ile Ala Gln Leu Lys Gln Lys
                   405                 410                 415
   aat gcc gca ctg aaa gag gaa att cag gcc ctg gaa tac ggc tca ggt     1296
   Asn Ala Ala Leu Lys Glu Glu Ile Gln Ala Leu Glu Tyr Gly Ser Gly
               420                 425                 430
   cca ggt tct cct gag gac aaa atc gca caa tta aaa gaa gaa aac caa     1344
   Pro Gly Ser Pro Glu Asp Lys Ile Ala Gln Leu Lys Glu Glu Asn Gln
           435                 440                 445
   cag ctg gaa cag aaa att caa gcc tta aaa gaa gag aac gct gcc ctg     1392
   Gln Leu Glu Gln Lys Ile Gln Ala Leu Lys Glu Glu Asn Ala Ala Leu
       450                 455                 460
   gaa tat ggt tct ggc cct ggc gat atc gaa caa gaa tta gaa aga gcc     1440
   Glu Tyr Gly Ser Gly Pro Gly Asp Ile Glu Gln Glu Leu Glu Arg Ala
   465                 470                 475                 480
   aaa gcc agt atc cgt cgt tta gaa caa gag gta aac caa gag cgt tca     1488
   Lys Ala Ser Ile Arg Arg Leu Glu Gln Glu Val Asn Gln Glu Arg Ser
                   485                 490                 495
   cgt atg gca tat tta cag aca ctg ctg gcc aaa agt ggt cct ggc tcc     1536
   Arg Met Ala Tyr Leu Gln Thr Leu Leu Ala Lys Ser Gly Pro Gly Ser
               500                 505                 510
   cca gag gat aaa aat gcc gct tta aaa gag gaa atc caa gcc tta gaa     1584
   Pro Glu Asp Lys Asn Ala Ala Leu Lys Glu Glu Ile Gln Ala Leu Glu
           515                 520                 525
   gag gaa aac cag gcg tta gag gaa aag att gct cag ctg aag tat ggt     1632
   Glu Glu Asn Gln Ala Leu Glu Glu Lys Ile Ala Gln Leu Lys Tyr Gly
       530                 535                 540
   tcc gga act agt ggt acc gta tcc aaa ggt gaa gag ctg ttt act ggt      1680
   Ser Gly Thr Ser Gly Thr Val Ser Lys Gly Glu Glu Leu Phe Thr Gly
   545                 550                 555                 560
   gtt gta ccg att ctg gtt gaa ctg gac ggt gac gta aat ggc cac aag      1728
   Val Val Pro Ile Leu Val Glu Leu Asp Gly Asp Val Asn Gly His Lys
                   565                 570                 575
   ttt tcc gtg tct ggc gag ggt gaa ggc gac gcg acc tac ggt aaa ctg      1776
   Phe Ser Val Ser Gly Glu Gly Glu Gly Asp Ala Thr Tyr Gly Lys Leu
               580                 585                 590
   acc ctg aaa ttc atc tgt act act ggc aaa ctg ccg gtt ccg tgg cca      1824
   Thr Leu Lys Phe Ile Cys Thr Thr Gly Lys Leu Pro Val Pro Trp Pro
           595                 600                 605
   acc ctg gtg acc acc ttc ggc tat ggc ctg caa tgt ttc gcg cgt tac      1872
   Thr Leu Val Thr Thr Phe Gly Tyr Gly Leu Gln Cys Phe Ala Arg Tyr
       610                 615                 620
   ccg gac cac atg aag cag cac gac ttc ttc aag tcc gcg atg cca gag      1920
   Pro Asp His Met Lys Gln His Asp Phe Phe Lys Ser Ala Met Pro Glu
   625                 630                 635                 640
   ggc tat gtt cag gaa cgc acc att ttt ttc aag gat gat ggt aac tac      1968
   Gly Tyr Val Gln Glu Arg Thr Ile Phe Phe Lys Asp Asp Gly Asn Tyr
                   645                 650                 655
   aag acc cgc gct gaa gtg aaa ttc gaa ggc gac acc ctg gtt aac cgc      2016
   Lys Thr Arg Ala Glu Val Lys Phe Glu Gly Asp Thr Leu Val Asn Arg
               660                 665                 670
   att gag ctg aaa ggc att gac ttc aaa gag gac ggt aac att ctg ggc      2064
   Ile Glu Leu Lys Gly Ile Asp Phe Lys Glu Asp Gly Asn Ile Leu Gly
           675                 680                 685
   cac aaa ctg gaa tat aat tac aat tcc cat aac gtc tat att atg gcc      2112
   His Lys Leu Glu Tyr Asn Tyr Asn Ser His Asn Val Tyr Ile Met Ala
       690                 695                 700
<210> 22
   <211> 704
   <212> PRT
   <213> Synthetic sequence
<220>
   <223> TET12 split YFP, tetrahedron-forming polypeptide sequence bound to
   split-YFP via C-terminal and N-terminal, respectively
<400> 22
   Asp Lys Gln Lys Asn Gly Ile Lys Val Asn Phe Lys Ile Arg His Asn
   1               5                   10                  15
   Ile Glu Asp Gly Ser Val Gln Leu Ala Asp His Tyr Gln Gln Asn Thr
               20                  25                  30
   Pro Ile Gly Asp Gly Pro Val Leu Leu Pro Asp Asn His Tyr Leu Ser
           35                  40                  45
   Tyr Gln Ser Ala Leu Ser Lys Asp Pro Asn Glu Lys Arg Asp His Met
       50                  55                  60
   Val Leu Leu Glu Phe Val Thr Ala Ala Gly Ile Thr Leu Gly Met Asp
   65                  70                  75                  80
   Glu Leu Tyr Lys Met Lys Gln Leu Glu Lys Glu Leu Lys Gln Leu Glu
                   85                  90                  95
   Lys Glu Leu Gln Ala Ile Glu Lys Gln Leu Ala Gln Leu Gln Trp Lys
               100                 105                 110
   Ala Gln Ala Arg Lys Lys Lys Leu Ala Gln Leu Lys Lys Lys Leu Gln
           115                 120                 125
   Ala Ser Gly Pro Gly Ser Pro Glu Asp Lys Ile Ala Gln Leu Lys Glu
       130                 135                 140
   Lys Asn Ala Ala Leu Lys Glu Lys Asn Gln Gln Leu Lys Glu Lys Ile
   145                 150                 155                 160
   Gln Ala Leu Lys Tyr Gly Ser Gly Pro Gly Asp Ile Glu Gln Glu Leu
                   165                 170                 175
   Glu Arg Ala Lys Ala Ser Ile Arg Arg Leu Glu Gln Glu Val Asn Gln
               180                 185                 190
   Glu Arg Ser Arg Met Ala Tyr Leu Gln Thr Leu Leu Ala Lys Ser Gly
           195                 200                 205
   Pro Gly Gln Leu Glu Asp Lys Val Glu Glu Leu Leu Ser Lys Asn Tyr
       210                 215                 220
   His Leu Glu Asn Glu Val Ala Arg Leu Lys Lys Leu Val Gly Ser Gly
   225                 230                 235                 240
   Pro Gly Met Lys Gln Leu Glu Lys Glu Leu Lys Gln Leu Glu Lys Glu
                   245                 250                 255
   Leu Gln Ala Ile Glu Lys Gln Leu Ala Gln Leu Gln Trp Lys Ala Gln
               260                 265                 270
   Ala Arg Lys Lys Lys Leu Ala Gln Leu Lys Lys Lys Leu Gln Ala Ser
           275                 280                 285
   Gly Pro Gly Ser Pro Glu Asp Glu Ile Gln Ala Leu Glu Glu Lys Asn
       290                 295                 300
   Ala Gln Leu Lys Gln Glu Ile Ala Ala Leu Glu Glu Lys Asn Gln Ala
   305                 310                 315                 320
   Leu Lys Tyr Gly Ser Gly Pro Gly Gln Leu Glu Asp Lys Val Glu Glu
                   325                 330                 335
   Leu Leu Ser Lys Asn Tyr His Leu Glu Asn Glu Val Ala Arg Leu Lys
               340                 345                 350
   Lys Leu Val Gly Ser Gly Pro Gly Ser Pro Glu Asp Lys Ile Ala Gln
           355                 360                 365
   Leu Lys Gln Lys Ile Gln Ala Leu Lys Gln Glu Asn Gln Gln Leu Glu
       370                 375                 380
   Glu Glu Asn Ala Ala Leu Glu Tyr Gly Ser Gly Pro Gly Ser Pro Glu
   385                 390                 395                 400
   Asp Glu Asn Ala Ala Leu Glu Glu Lys Ile Ala Gln Leu Lys Gln Lys
                   405                 410                 415
   Asn Ala Ala Leu Lys Glu Glu Ile Gln Ala Leu Glu Tyr Gly Ser Gly
               420                 425                 430
   Pro Gly Ser Pro Glu Asp Lys Ile Ala Gln Leu Lys Glu Glu Asn Gln
           435                 440                 445
   Gln Leu Glu Gln Lys Ile Gln Ala Leu Lys Glu Glu Asn Ala Ala Leu
       450                 455                 460
   Glu Tyr Gly Ser Gly Pro Gly Asp Ile Glu Gln Glu Leu Glu Arg Ala
   465                 470                 475                 480
   Lys Ala Ser Ile Arg Arg Leu Glu Gln Glu Val Asn Gln Glu Arg Ser
                   485                 490                 495
   Arg Met Ala Tyr Leu Gln Thr Leu Leu Ala Lys Ser Gly Pro Gly Ser
               500                 505                 510
   Pro Glu Asp Lys Asn Ala Ala Leu Lys Glu Glu Ile Gln Ala Leu Glu
           515                 520                 525
   Glu Glu Asn Gln Ala Leu Glu Glu Lys Ile Ala Gln Leu Lys Tyr Gly
       530                 535                 540
   Ser Gly Thr Ser Gly Thr Val Ser Lys Gly Glu Glu Leu Phe Thr Gly
   545                 550                 555                 560
   Val Val Pro Ile Leu Val Glu Leu Asp Gly Asp Val Asn Gly His Lys
                   565                 570                 575
   Phe Ser Val Ser Gly Glu Gly Glu Gly Asp Ala Thr Tyr Gly Lys Leu
               580                 585                 590
   Thr Leu Lys Phe Ile Cys Thr Thr Gly Lys Leu Pro Val Pro Trp Pro
           595                 600                 605
   Thr Leu Val Thr Thr Phe Gly Tyr Gly Leu Gln Cys Phe Ala Arg Tyr
       610                 615                 620
   Pro Asp His Met Lys Gln His Asp Phe Phe Lys Ser Ala Met Pro Glu
   625                 630                 635                 640
   Gly Tyr Val Gln Glu Arg Thr Ile Phe Phe Lys Asp Asp Gly Asn Tyr
                   645                 650                 655
   Lys Thr Arg Ala Glu Val Lys Phe Glu Gly Asp Thr Leu Val Asn Arg
               660                 665                 670
   Ile Glu Leu Lys Gly Ile Asp Phe Lys Glu Asp Gly Asn Ile Leu Gly
           675                 680                 685
   His Lys Leu Glu Tyr Asn Tyr Asn Ser His Asn Val Tyr Ile Met Ala
       690                 695                 700

## Description

### Field of invention

The present invention relates to polypeptides capable of forming polyhedra by self-assembly, polyhedra formed of polypeptides, fusion polypeptides of polyhedra-forming polypeptides with functional polypeptides, DNA coding for such polypeptides or fusion polypeptides, and a method for forming polypeptide polyhedra from such polypeptides. Disclosure is made of a method of treating or diagnosing a human or an animal using such polypeptides and the use of such polypeptides in different applications.

### Background of the invention

Recently, DNA manipulation has been used to build artificial structures at the nanometric scale relying on the simple base-pairing properties of DNA duplex. Designed nanostructures composed of intertwined complementary nucleotide segments represent the building blocks that build up the structure. Structures built from DNA include geometric objects, such as tetrahedron, cube, octahedron, dodecahedron or buckyball as well as two dimensional tetragonal or hexagonal lattice (He, Ye, Su, Zhang, Ribbe, Jiang and Mao, Nature, 452:198-202, 2008), and different patterns obtained by DNA origami.

### Description of the disclosure made and the invention

The following disclosure is made:
1. Polypeptide capable of forming a polyhedron by self-assembly, wherein the polypeptide contains at least 12 coiled-coil-forming segments connected via linker segments, wherein each of the edges of the polyhedron is constructed of a pair of said coiled-coil-forming segments and each pair of said coiled-coil-forming segments forms a coiled-coil structure.

Preferably, the polypeptide forms a closed path along the edges of the polyhedron. Further, the linker segments are preferably nonhelical linker segments.

Structures and functional devices in nature are predominantly based on polypeptides and are far more complex and technologically applicable than those based on nucleic acids. Polypeptides are more stable than nucleic acids and have versatile chemical properties due to the availability of twenty different amino acids in comparison to only four different nucleotides. Therefore using polypeptides for self-assembled nanostructures represents an advantage that can endow the prepared nanomaterial with additional technological properties which include increased stability, versatility of tertiary structures, creation of catalytic sites, engineering of specific interaction/recognition sites, introduction of specific properties such as optical, electrical and mechanical properties.

Each pair of coiled-coil-forming segments forms a coiled-coil structure, which is a predominantly linear and rigid structure, thus very suited for forming an edge of a polyhedron. By provision of a polypeptide folding to form a polyhedron, it is possible to obtain a polyhedron structure having increased stability compared to DNA polyhedra. Similarly, by providing a polypeptide composed of a single chain, stability is increased compared to polypeptide polyhedra formed of multiple (non-connected) polypeptides due to the covalent binding between the segments.

A polyhedron has a defined structure and hydrodynamic diameter while being highly symmetric. Thus, these structures can be easily purified or separated due to their highly uniform physical and chemical properties. Moreover, polyhedra provide a specified three-dimensional shape, which is important for the creation of structures and materials. Furthermore, polypeptide polyhedra allow precise three-dimensional positioning of functional groups, which is important for the creation of catalytic sites, molecular recognition, and creation of chemical as well as physical properties in general.

In addition, it is possible to encage substances, such as drugs, inside the polyhedral structure of the polypeptide (nanocage). Polyhedra enclose a defined volume, which is particularly important for the delivery of drugs or other substances. Thus, it is possible to administer drugs to a patient or animal, in particular drugs which would not be tolerated if administered without protection by the polyhedral structure. Once the nanocage containing the substance has reached its target position, it can be opened up either by natural degradation, thus allowing a slow release of the drug (depot function), or by externally triggering a break of the polypeptide structure (e.g. light-induced breakage of the polypeptide chain caused by a substance linked to the side chain of an amino acid residue of a linker segment).
2. Polypeptide according to item 1, wherein each coiled-coil-forming segment of a pair of coiled-coil-forming segments has higher probability of forming a coiled-coil structure with the other coiled-coil-forming segment of said pair of coiled-coil-forming segments than with any other coiled-coil-forming segment of the polypeptide.
   By requiring a specific binding between each pair of coiled-coil-forming segments exactly once per molecule, the formation of the desired polyhedral structure can be assured. If a specific pair of coiled-coil-forming peptide sequences is present more than once per molecule, this could lead to undesired folding. In this case, the position of the respective pairs has to be selected such that no undesired folding can take place e.g. due to steric hindrance.
3. Polypeptide according to item 1 or 2, wherein the nonhelical linker segments are flexible linker segments.
   The use of flexible linkers facilitates the self-assembly by allowing all angles between neighboring coiled-coil-forming peptide sequences.
4. Polypeptide according to one of items 1 to 3, wherein the nonhelical linker segments each consist of at least 1 amino acid residues, preferably 1 to 10 amino acid residues, more preferably 2 to 6 amino acid residues, most preferably 4 amino acid residues.
   If the linker segment is too short, it does not provide a sufficient degree of freedom to allow the formation of the polyhedral structure, e.g. because of steric hindrance or angular restrictions. If the linker segment is too long, it may be too flexible and allow undesired folding or may form a secondary structure with neighboring linker segments, thus possibly preventing the formation of the desired polyhedral structure.
5. Polypeptide according to one of items 1 to 4, wherein the linker contains hydrophilic amino acid residues capable of breaking α-helical structure.
   Thus, it is assured that the linker can act as a linker without interference with the coiled-coil-forming segments, i.e. without requiring a breaking of a continued helix formed by the linker segment upon formation of the polyhedron.
6. Polypeptide according to one of items 1 to 5, wherein the linker contains at least 50% by number of amino acid residues selected from the group consisting of glycine, serine threonine, proline, cysteine, histidine, tryptophane, and tyrosine.
   A high number of these residues, i.e. at least half of the number of residues forming the linker segment, provides an increased probability that the linker does not form a secondary structure, in particular continues the formation of a helical structure. The number of residues selected form this list is preferably 75% of all residues constituting the linker segment.
7. Polypeptide according to one of items 1 to 6, wherein the nonhelical linker segments are tetrapeptide segments with the amino acid sequence SGPG.
   In the tests, this linker segment has been shown to provide very good properties.
8. Polypeptide according to one of items 1 to 7, wherein the polypeptide contains at least one of the coiled-coil-forming segments represented by SEQ ID NOs. 2, 4, 6, 8, 10, 12, 14, 16 and 18.
   The used coiled-coil-forming segments allow the formation of a polyhedron with a low probability of misfolding due to their specific and orthogonal binding properties, i.e. strong binding to the desired binding partner and weak binding to other potential binding partners.
9. Polypeptide according to one of items 1 to 8, wherein the polypeptide contains at least one coiled-coil-forming segment derived from naturally occurring leucine-zipper proteins.
   These naturally occurring coiled-coil-forming segments can be used to create larger structures e.g. in combination with the above-mentioned designed coiled-coil-forming segments.
10. Polypeptide according to one of items 1 to 9, wherein the polyhedron is a tetrahedron.
   The tetrahedron is the simplest polyhedron and thus facilitates the process of self-assembly and further requires the least number of coiled-coil-forming segments. Consequently, the probability of misfolding is reduced.
11. Polypeptide according to item 10, wherein the polypeptide consist of 12 coiled-coil-forming segments.
   A number of twelve coiled-coil-forming segments is sufficient to form a tetrahedron. Any additional pair of coiled-coil-forming segments would only result in an antenna-like extension from the tetrahedron, provided that the structure is designed to form a polyhedron. Moreover, additional coiled-coil-forming segments decrease the binding selectivity between designated pairs of coiled-coil-forming polypeptide sequences, thus requiring more moderate conditions for the self-assembly.
12. Polypeptide according to item 10 or 11, wherein two out of the six edges of the tetrahedron are formed of antiparallel coiled-coils and four are composed of parallel coiled-coil dimers.
   This option is one out of two possibilities of forming a tetrahedron from coiled-coil-forming segments.
13. Polypeptide according to item 12, wherein the sequential order of antiparallel coiled-coil-forming segments A and parallel coiled-coil-forming segments P is APAP PPPP APAP or one of its cyclic permutations.
14. Polypeptide according to item 12 or 13, wherein the sequential order of coiled-coil-forming segments is Aa-Pa-Ab-Pb-Aa'-Pc-Pb'-Pa'-Pd-Pc'-Ab'-Pd' or a cyclic permutation thereof, wherein Aa and Ab represent coiled-coil-forming segments forming antiparallel coiled-coils with Aa' and Ab', respectively, and Pa to Pd represent coiled-coil-forming segments forming parallel coiled-coils with Pa' to Pd', respectively.
   These structures allow formation of a stable self-assembling tetrahedron.
15. Polypeptide according to one of items 12 to 14, wherein the sequential order of coiled-coil-forming segments is APH-P3-BCR-GCN-APH-P7-GCN-P4-P5-P8-BCR-P6 represented by SEQ ID NOs 2, 8, 4, 6, 2, 16, 6, 10, 12, 18, 4, and 14, respectively, or a cyclic permutation thereof.
   In the examples, this structure has been shown to provide a reliable formation of a tetrahedral structure. Further, the coiled-coil-forming segments APH-APH and BCR-BCR can be switched, and P3-P4, P5-P6, P7-P8 and GCN-GCN can be switched while maintaining the suitability for the formation of a tetrahedron. For example, in a sequence containing P3 and P4 as a pair of coiled-coil-forming segments, P3 can be switched with P7 (or P8) if P4 is at the same time switched with P8 (or P7).
16. Polypeptide according to item 10 or 11, wherein three out of six edges of the tetrahedron are composed of antiparallel coiled-coil dimers and three of parallel coiled-coil dimers.
   This option is the second out of two possibilities of forming a tetrahedron from coiled-coil forming polypeptide sequences.
17. Polypeptide according to one of items 1 to 16, wherein the coiled-coil-forming segments are composed of n heptad patterns a-b-c-d-e-f-g, which may be the same or different for each heptad, and n is preferably in the range of from 3 to 50.
   Thus, the size of the polyhedron can be suitably adjusted.
18. Polypeptide according to item 19, wherein the residues at positions "b", "c" and/or "f" of the heptads of coiled-coil-forming segments are modified so that they endow additional functions to the self-assembling polypeptide polyhedron.
   Since these residues are only required to maintain the secondary structure, a suitable modification can be added to these residues without affecting the tertiary structure.
19. Polypeptide according to item 18, wherein the additional function is one or more selected out of the group consisting of net charge, polarity, metal chelation, binding sites for drugs, dyes, catalytic centres, antigenic determinants, optical properties.
   Thus, a desired function can be added to the polypeptide.
20. Fusion polypeptide of a polyhedron-forming polypeptide according to one of items 1 to 19 with a functional polypeptide domain attached to the N- and/or C-terminal segment of the polyhedron-forming polypeptide.
   By this, a desired functionality provided by a polypeptide can be added to the polyhedron-forming polypeptide. Such a functionality is easily accessible via bimolecular routes (e.g. expression in a cell).
21. Fusion polypeptide according to item 20, wherein the functional polypeptide domain is a non-split functional polypeptide domain.
   Using a non-split functional polypeptide domain allows the domain being active independently of the actual state of folding of the self-assembling polyhedron.
22. Fusion polypeptide according to item 20, wherein the functional polypeptide domain is split into two segments that are linked to the N-terminus and C-terminus of the polypeptide, respectively, wherein the two segments of the functional polypeptide domain reassemble upon formation of a tetrahedron and gain function.
   A functional polypeptide which is split into two segments and gains function upon self-assembly of the polyhedral structure allows triggering the function of this functional polypeptide. Moreover, the successful formation of the polyhedral structure can be confirmed by confirming the gain of function.
23. Fusion polypeptide according to item 22, wherein the functional polypeptide domain is a fluorescent protein split into two segments and the segments are attached to the N- and C-terminal end of the polyhedron-forming polypeptide, respectively.
   Thus, the formation of the polyhedral structure can be confirmed by fluorescence spectroscopy. Accordingly, it can be confirmed, for example, that encaging of a target substance has been successful or that the nanocage formed by the polypeptide is still faultless.
24. Polypeptide polyhedron formed from a polypeptide according to one of items 1 to 19 or a fusion polypeptide according to one of items 20 to 23.
   This is the result of self-assembly of the polyhedron-forming polypeptide of the invention. The present invention further relates to a polypeptide polyhedron according to item 25:
25. Polypeptide polyhedron, whose edges are composed of linked coiled-coil-forming dimeric segments, wherein the number of said segments is twice the number of the edges of the polyhedron, wherein the polypeptide forms a closed path along the edges of the polyhedron and nonhelical linker segments are located between the coiled-coil-forming dimeric segments and form the vertices of the polyhedron.
26. DNA coding for a polypeptide according to one of items 1 to 19, a fusion polypeptide according to one of items 20 to 23, or a polypeptide polyhedron according to item 24 or 25.
   Using such DNA, the polypeptide of the invention can be easily achieved.
27. DNA according to item 26 further comprising regulatory elements for the production of the polypeptide or fusion polypeptide in living cells, preferably bacteria, or by in vitro transcription/translation.
   The regulatory elements are usual in the art and facilitate the production of the desired polypeptide in high amounts.
28. Method of forming a polypeptide polyhedron, wherein the environment of a polypeptide according to one of items 1 to 19 or a fusion polypeptide according to one of items 20 to 23 is exchanged from an unfolding condition for the polypeptide to a folding condition for the polypeptide.
   In the unfolding condition, none of the coiled-coil structures is formed, so that an open structure is present. Usually, only few secondary structure elements will be present in this state (random coil structure). Upon exchange to the folding condition, in which the coiled-coil structure elements start forming, the self-assembly of the polyhedron proceeds.
29. Method according to item 28, wherein the time duration for exchanging from the unfolding condition to the folding condition is at least 16 hours, preferably at most 40 hours.
   An increased time for allowing the system to reach the equilibrium state prohibits the formation of undesired folding (misfolding). Usually, 40 hours are sufficient so that a further increase of time would not bring much more benefit.
30. Method according to item 28 or 29, wherein the exchange from the unfolding condition to the folding condition is a continuous exchange.
   A continuous exchange is preferable due to the possibility to be close to the equilibrium state at any point of time.
31. Method according to item 28 or 29, wherein the exchange from the unfolding condition to the folding condition is a stepwise exchange having at least 5 step, preferably from 5 to 10 steps.
   A stepwise exchange can be accomplished more easily from a technical point of view.
32. Method according to one of items 28 to 31, wherein the unfolding condition is represented by a concentration of a chemical denaturing agent which is sufficient to break up the coiled-coil structure of the most stable pair of coiled-coil-forming segments and the folding condition is represented by a concentration of a chemical denaturing agent which is less than required to break up the coiled-coil structure of the least stable pair of coiled-coil-forming segments.
   Chemical denaturing agents are well known and can be applied easily.
33. Method according to one of items 28 to 32, wherein the unfolding condition is represented by a temperature which is sufficient to break up the coiled-coil structure of the most stable pair of coiled-coil-forming segments and the folding condition is represented by a temperature which is less than required to break up the coiled-coil structure of the least stable pair of coiled-coil-forming segments.
   Thermal denaturing methods are often employed in the art and are suitable for large scale production due to a low amount of by-products and ease of purification.
34. Method for treating or diagnosing a human or an animal by encaging a drug into a polyhedral nanocage formed from a polypeptide according to one of items 1 to 19 or fusion polypeptide according to one of items 20 to 23 and administering the polyhedral nanocage to the human or animal.
   The nanocage formed by the polyhedron can be used to deliver a drug into a body.
36. Method according to item 35, wherein the polypeptide contains target-specific binding substances directing the polyhedral nanocage to a desired tissue. Thus, site-specific drug delivery is possible.
37. Use of a polypeptide according to one of items 1 to 19, a fusion polypeptide according to one of items 20 to 23, or a polypeptide polyhedron according to item 24 or 25 for drug delivery, for chemical catalysis, as a carrier of vaccines, antitumor agents or other pharmaceutical agents, for the assembly of inorganic or organic compounds at defined spatial positions of the polypeptide polyhedral scaffold, or for diagnostics. 38. Use according to item 37, wherein the diagnostics are based on the self-assembly or reconstitution of split protein domains inserted at selected positions within the polyhedral polypeptide structure.

The invention to which the present specification pertains is set out in the claims appended to this description.

### Description of the drawings

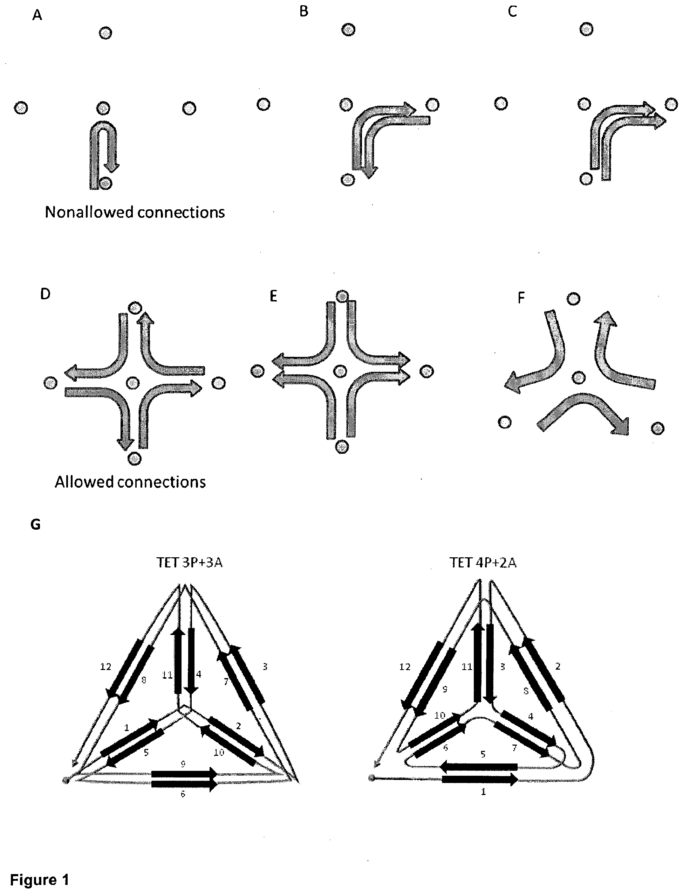

**Figure 1****:** (A,B,C) Nonallowed connections between segments forming the edges of a polyhedron shown on an example of a vertex converging four edges, which however is also relevant for vertices of other types. (D,E,F) Example of two types of allowed connections in the vertices converging four edges (D,E) or three edges (F). (G) Two possibilities in design of polypeptide tetrahedron: one includes 3 antiparallel and 3 parallel coiled-coils, the other one includes 2 antiparallel and 4 parallel coiled-coils.

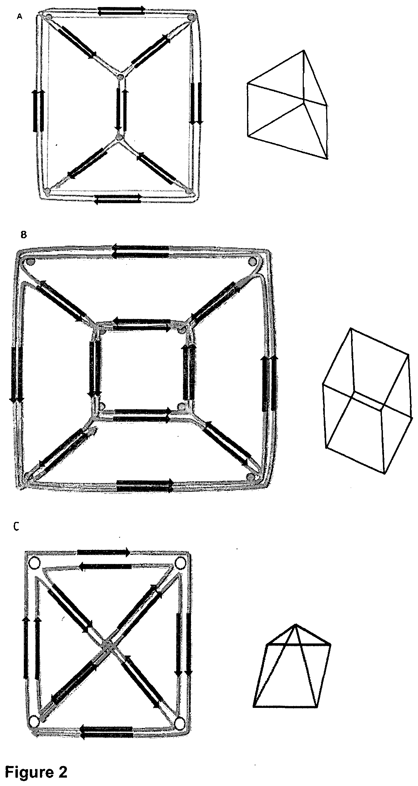

**Figure 2****:** Scheme of three examples of polyhedra composed of a single polymer chain: (A) trigonal prism, (B) tetragonal prism and (C) tetragonal pyramid.

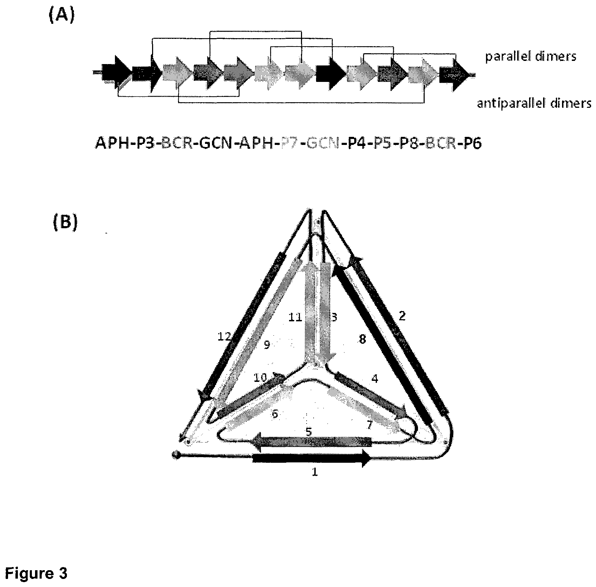

**Figure 3****: (A)** Scheme of the polypeptide chain consisting of twelve coiled-coil-forming segments. **(B)** Scheme of the assembly of polypeptide chain comprising twelve coiled-coil-forming segments, where six of them form parallel heterodimers (2-8, 6-10, 9-12), two of them form parallel homodimer (4-7), and four of them form antiparallel homodimers (1-5, 3-11), can self-assemble into the tetrahedron. Arrows denote the orientation of the interacting coiled-coil-forming segments in the assembly.

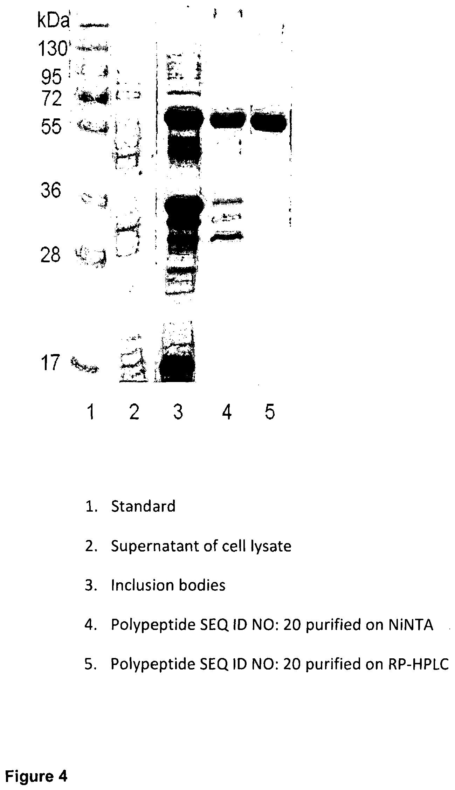

**Figure 4****: (A)** Isolation of polypeptide SEQ ID NO: 20 after its expression with DNA construct SEQ ID NO: 19: after cell lysis supernatant (lane 2) and insoluble fraction (inclusion bodies, lane 3) were checked for the presence of polypeptide. The polypeptide was mainly obtained in form of inclusion bodies (lane 3), and was purified with affinity chromatography on Ni-NTA column (line 4) and further with HPLC-RP chromatography (lane 5).

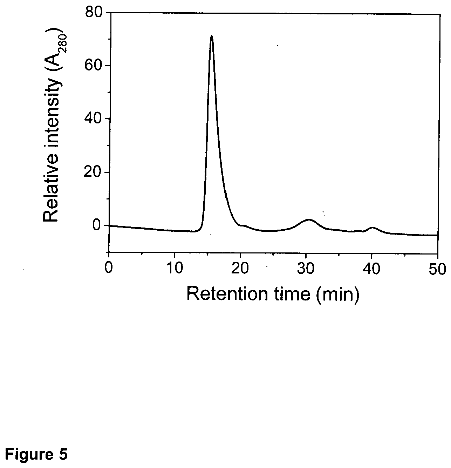

**Figure 5****:** The purity of the isolated polypeptide SEQ ID NO: 20 was demonstrated by size exclusion chromatography using BioSep s2000 column (Phenomenex). Separation was performed in the presence of 6 M GdnHCl.

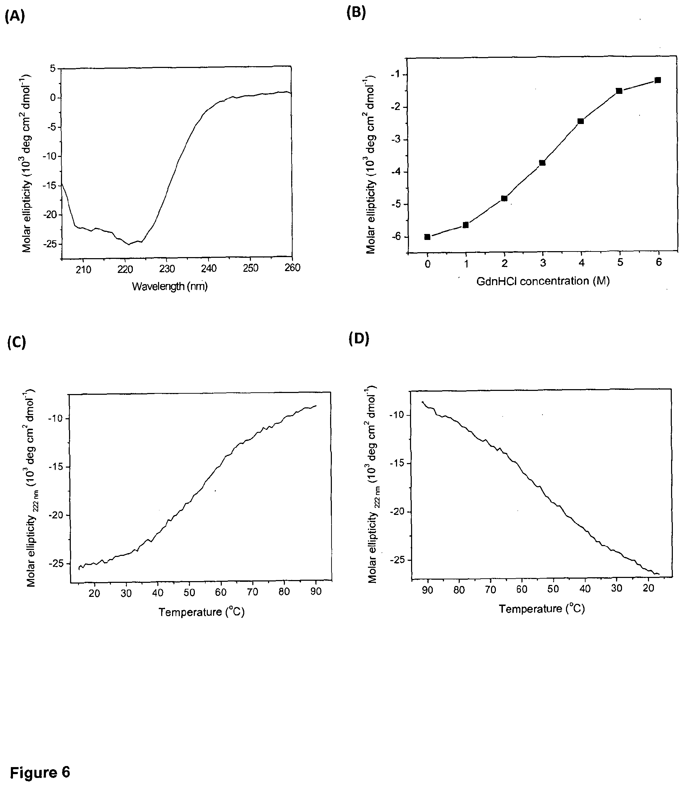

**Figure 6****:** Circular dichroism (CD) measurements. **(A)** Secondary structure of the assembled polypeptide SEQ ID NO: 20 was determined by measuring circular dichroism in the far UV range, demonstrating that the coiled-coils are formed. **(B)** Chemical denaturation curve was obtained by measuring the CD₂₂₂ₙₘ signal for polypeptide SEQ ID NO: 20 in solutions with GdnHCl concentration between 0 M and 6 M. The midpoint of transition is at 3.2 M GdnHCl. The thermal stability was determined by heating **(C)** and cooling **(D)** the polypeptide SEQ ID NO: 20 solution at concentration of 0.2 mg/ml. The midpoint of reversible thermal transition at 56°C was calculated.

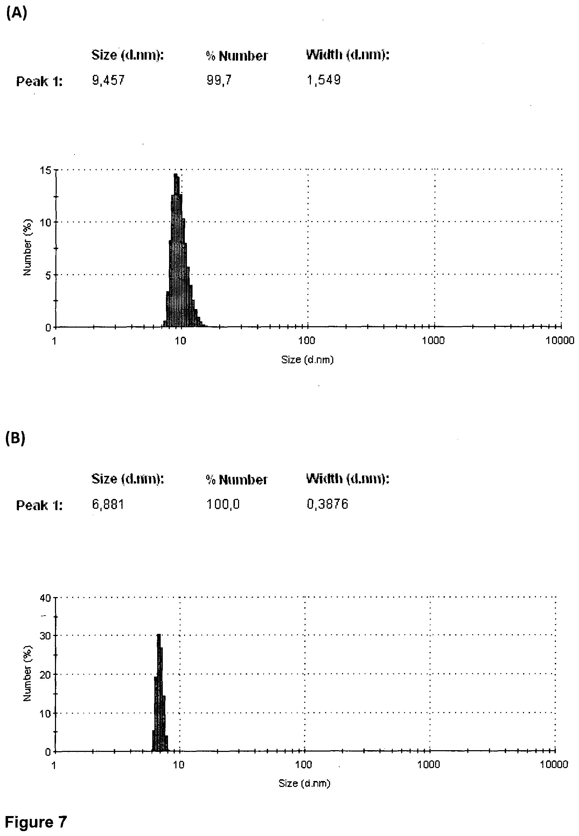

**Figure 7****:** Dynamic light scattering (DLS) measurement determination of the size distribution of polypeptide SEQ ID NO: 20 particles in solution. In the presence of 6 M GdnHCl in the buffer the hydrodynamic diameter of denatured polypeptide is 9.5 nm (A), whereas polypeptide self-assembled under native conditions had a hydrodynamic diameter of 6.9 nm (B).

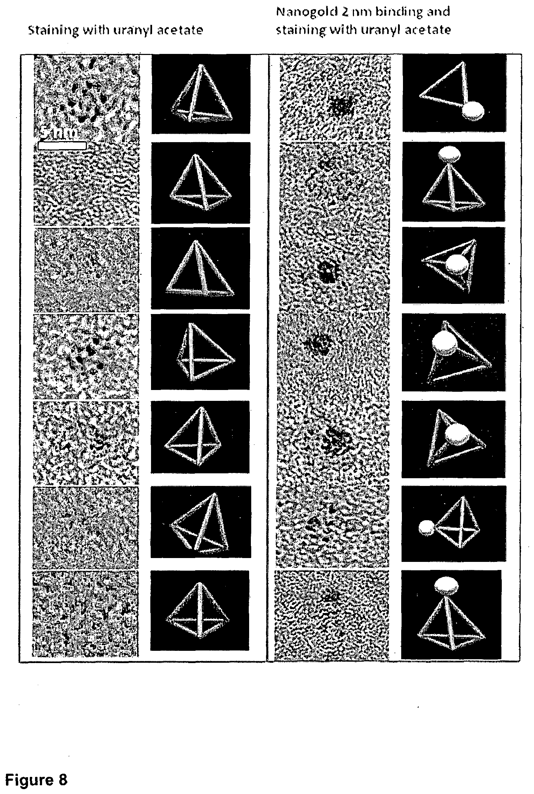

**Figure 8****:** Transmission electron microscopic image (TEM) of the assembled polypeptide SEQ ID NO: 20 (self-assembled over 16 hours from 6 M to 0 M GdnHCl at polypeptide concentration of 5 µg/mL). Sample was stained with nanogold of 2 nm (shown as sphere), which marked His tags at one vertex of the tetrahedron, following by uranyl acetate staining and then visualized on TEM. The assembled tetrahedra were appropriate dimensions.

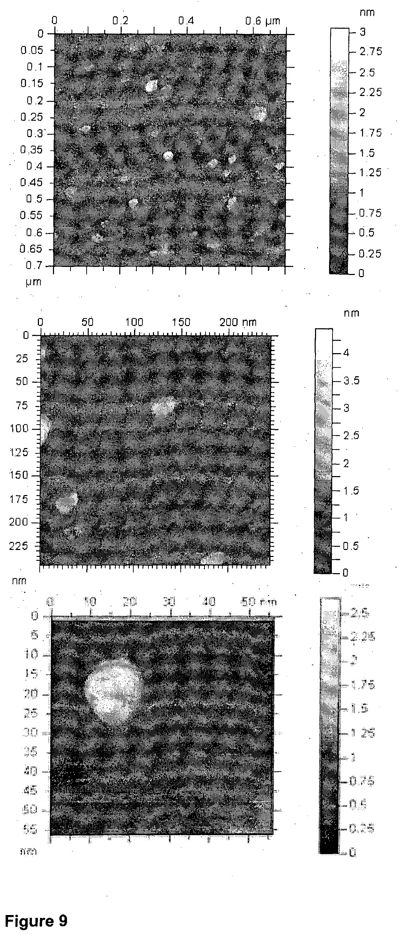

**Figure 9****:** AFM scan of the assembled polypeptide SEQ ID NO: 20 (self-assembled over 16 hours of decreasing the concentration from 6 M to 0 M GdnHCl at polypeptide concentration of 5 µg/mL). Sample was deposited on mica and an image recorded by Agilent Technologies 5500 Scanning Probe Microscope operating in Acoustic alternative current AFM mode using silicon cantilever PPP-NCH with force constant 42 N/m and tip radius less than 7nm (Nanosensors).

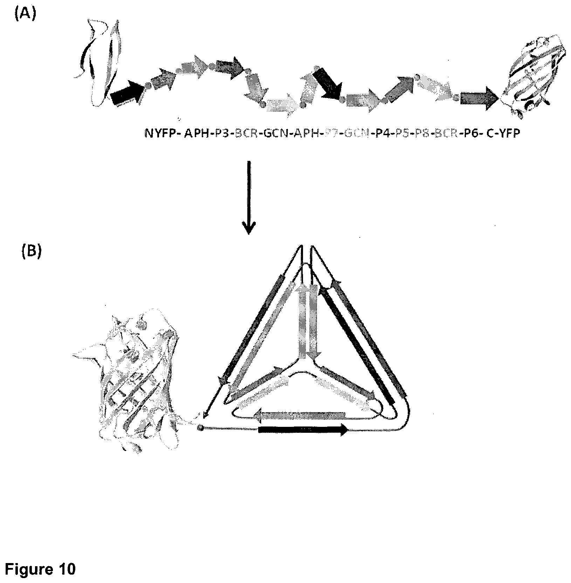

**Figure 10****: (A)** Scheme of the polypeptide SEQ ID NO: 22 chain consisting of twelve coiled-coil-forming segments with additional split yellow fluorescence protein (YFP) segments coupled at each of the ends based on the synthetic gene for the fusion polypeptide. (B) Scheme of the assembly of polypeptide chain described above. Only the regular assembly enables the reconstruction of the protein split YFP and its fluorescence.

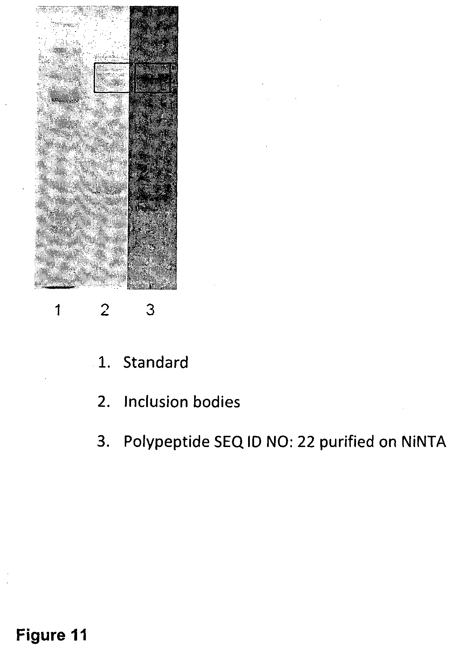

**Figure 11****:** Isolation of polypeptide SEQ ID NO: 22 after its expression with DNA construct SEQ ID NO: 21: after cell lysis insoluble fraction (inclusion bodies, lane 2) were checked for the presence of a polypeptide. The polypeptide was purified from inclusion bodies with affinity chromatography on Ni-NTA column (line 3).

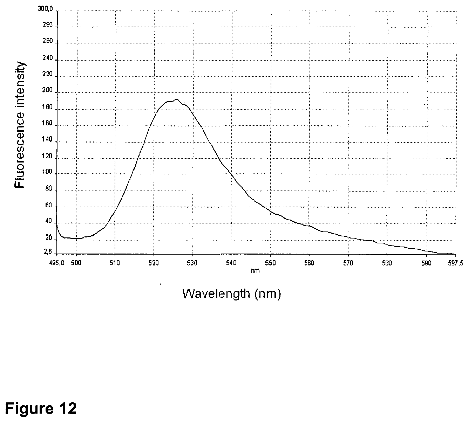

**Figure 12****:** Fluorescence measurement after the self-assembly of the polypeptide SEQ ID NO: 22. The regular assembly enabled the reconstruction of split YFP whose fluorescence was detected.

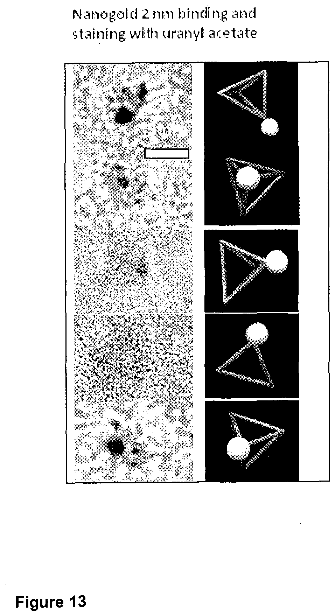

**Figure 13****:** TEM image of the self-assembled polypeptide SEQ ID NO: 22. Sample was stained with nanogold of 2 nm (shown as sphere), which binds to hexahistidine peptide tags at one vertex of the tetrahedron, followed by uranyl acetate staining and then visualization on TEM. The assembled tetrahedra had the expected dimensions.

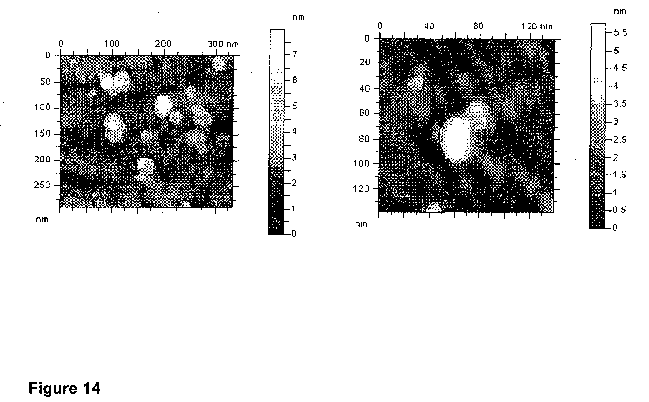

**Figure 14****:** AFM scan of the self-assembled polypeptide SEQ ID NO: 22 (self-assembled over 16 hours from 6 M to 0 M GdnHCl at polypeptide concentration of 5 µg/mL) deposited on mica. Scan revealed the expected shape of the assembled structures, composed of the assembled tetrahedron and reconstructed YFP.

The benefits and embodiments of the invention will be more apparent from the following description making reference to the attached figures and describing the principles underlying the present invention in detail.

Polypeptide design represents a route to new structures and functions with potential properties unseen in nature. It requires reliable rules that link polypeptide sequence to structure/function. Since the polypeptide assembly is extremely complex as it involves a large number of cooperative interactions the inventors solved the folding problem by inventing a new principle of polypeptide nanostructure design, where the polypeptide is composed of connected rigid modular building blocks for which one can reliably predict the structure as well as pair-wise interactions within polypeptide assemblies. This provides that the polypeptide self-assembles into the designed polyhedron whose edges are formed of pairs of building blocks.

The coiled-coil is one of the simplest super-secondary-structure motif and one understands quite well the interactions governing the specificity of coiled-coil formation. Therefore, the α-helical coiled-coil is an attractive choice for using it as building block because the rules governing its structure, oligomerization state and partner specificity are the most developed for any protein-folding motif. Coiled-coil dimers can be assembled in either parallel or antiparallel orientation and can be formed either as heterodimers or homodimers, depending whether their binding partner is the same or different polypeptide, respectively. Coiled-coil-forming segments can be structurally dissected into a sequence of heptad patterns, where defined positions are occupied preferentially by hydrophobic (positions "a" and "d") and charged (positions "e" and "g") amino acid residues. Those residues participate in interactions with partner coiled-coil-forming segments. In addition, residues at positions "b", "c" and "f" participate only to the extent of their contribution to the helical propensity and can be used to engineer additional desired properties into the self-assembled structure.

One can treat the coiled-coil-forming segments as rigid rods that can be concatenated into a larger polypeptide chain by introducing a flexible linker between them. The defined order of connected coiled-coil-forming segments enables formation of structures based on predictable interactions among the segments. In this way it is possible to build different polyhedral structures where coiled-coil segments represent the edges of the assembled structure.

As presented by the inventors, polyhedra can be self-assembled from a single designed polypeptide chain which is composed of concatenated coiled-coil forming segments separated by short flexible linker peptides. The number of required coiled-coil forming segments is twice the number of edges of a polyhedron, while the edges are formed of coiled-coil dimers. The order and pairwise interactions between coiled-coil-forming segments and their path along the edges of a polyhedron have to be carefully selected by the rules presented in this invention for obtaining a pure polyhedral shape. On an example of a tetrahedron the inventors demonstrate that it can be self-assembled from a single polypeptide chain that is composed of twelve coiled-coil-forming segments connected with flexible linkers and the coiled-coil-forming segments form six coiled-coils which form the six edges the tetrahedron.

The invention specifically refers to a nanostructure self-assembled from a single polypeptide chain that comprises a defined sequence of coiled-coil-forming segments that form homodimeric or heterodimeric coiled-coil peptide pairs with their binding partner within the same molecule of the polypeptide chain. The coiled-coil-forming segments span preferentially between three to 50 heptads. The nanostructures formed according to the invention are polyhedra, which can be constructed from rigid edges. A specific embodiment of the polyhedron of this invention is a polypeptide tetrahedron.

In a preferred embodiment, the invention refers to a polypeptide that self-assembles into nanostructure named above, wherein the polypeptide comprises the following arrangement of twelve (12) coiled-coil-forming segments with the following sequential order of segments:
(1) antiparallel homodimer-forming segment APH - (2) parallel heterodimer-forming segment P3 - (3) antiparallel homodimer-forming segment BCR - (4) parallel homodimer-forming segment GCNshort - (5) antiparallel homodimer-forming segment APH - (6) parallel heterodimer-forming segment P7 - (7) parallel homodimer-forming segment GCNshort - (8) parallel heterodimer-forming segment P4 - (9) parallel heterodimer-forming segment P5 - (10) parallel heterodimer-forming segment P8 - (11) antiparallel homodimer-forming segment BCR - (12) parallel heterodimer-forming segment P6.

Each of antiparallel homodimer-forming segments APH and BCR form antiparallel homodimeric coiled-coils (APH-APH, BCR-BCR), and the parallel homodimer-forming segment GCNshort forms a parallel homodimeric coiled-coil (GCNshort-GCNshort). The segments APH, BCR, GCNshort form dimers exclusively with an identical segment, but not with other types of homodimer-forming segments; whereas parallel heterodimer-forming segments P3, P5, P7 form parallel heterodimeric coiled-coils exclusively with their partners P4, P6, P8, respectively.

The invention refers to the polypeptide as said above, wherein the coiled-coil-forming segments are selected among coiled-coil-forming segments of natural polypeptides or designed polypeptides and are preferentially selected among the peptides SEQ ID NOs: 2, 4,6,8,10,12,14,16 and 18.

The invention refers to a polypeptide as said above, wherein polypeptide linkers are added between coiled-coil-forming segments, where the length of the linker peptide is preferentially from one to 10 amino acid residues, more preferentially of four amino acid residues. A peptide tag, e.g. composed of six histidine amino acid residues, a Flag tag, a HA tag or a AU tag, may be added at the N- or C-terminus as well. This type of tag does not hinder the formation of coiled-coil connections and provides additional functional properties of polypeptide, such as feasibility of detection, purification, addition of optical properties and immunological, catalytic, charged or other chemical or biological activity. The polypeptide according to the invention may be prepared by recombinant method in bacteria or other generally used host production organism or by in vitro transcription/translation system.

The invention further refers to DNA coding for the polypeptide of the invention. The DNA is preferably operatively linked to the regulatory elements, promoter and terminator, which drive expression of fusion proteins in the host cells.

The disclosure further refers to a procedure for the assembly of a polypeptide into polyhedra named above. The process may include slow decrease of concentration or removal of a chemical denaturing agent, preferentially guanidinium hydrochloride, urea, lithium chloride or other chemical unfolding agents, where the procedure comprises setting the polypeptide in a state of unfolding by exposing it to previously determined concentration range of the denaturing agent and by slow solvent exchange during dialysis of the polypeptide from unfolding conditions employing a slow decrease of the concentration of the denaturing agent in a dialysis solution, which is preferably a continuous over between 16 and 40 hours, or by a stepwise decrease of the concentration of denaturing agent in a dialysis solution in preferably more than five, more preferably between five and ten steps. Alternatively slow temperature annealing can also be used for the polypeptide self-assembly. In this case, annealing starts at a temperature above the stability (dissociation temperature) of the most stable coiled-coil dimeric segment and decreasing the temperature below the stability of the least stable coiled-coil dimeric segment.

The invention refers to the application of a nanostructure tetrahedron according to the invention and application of the polypeptides of the invention as molecular carriers to encase other compounds that can be used for different applications, such as drug delivery, for chemical catalysis within the polypeptide polyhedral nano-cage or at the surface of the assembled nanostructure, for binding of specific compounds, as a carrier of vaccines, antitumor or other pharmaceutical agents, assembly of inorganic or organic compounds at the defined positions of the polypeptide polyhedral scaffold, for diagnostics, based on the self-assembly or reconstitution of split protein domains inserted at selected positions within the polyhedral polypeptide structure.

Unless defined otherwise, all technical and scientific terms used herein possess the same meaning as it is commonly known to experts in the field of invention. The terminology to be used in the description of the invention has the purpose of description of a particular segment of the invention and has no intention of limiting the invention. All publications mentioned in the description of the invention are listed as references. In the description of the invention and in the claims, the description is in the singular form, but also includes the plural form, which is not specifically highlighted for the ease of understanding.

In the past, design of structures from polypeptides has been limited to homologues of natural folds. This invention allows design of virtually any three-dimensional shape that can be tessellated.

The basis of invention is a surprising finding by inventors that only defined combinations of linked coiled-coil-forming segments within a single polypeptide chain can self-assemble into polypeptide polyhedra on the nanometer scale. Inventors describe the process for selection of these defined combinations. This is an important improvement in polypeptide planar or three-dimensional nanostructure design since combination of two coiled-coil-forming segments can lead only to the formation of linear, fibrillar assemblies (US 7,045,537 B1).

### Polypeptide assembly into polyhedron

The term "polyhedron" refers to the assembly of polypeptide chain that apparently forms the edges of a polyhedron with dimensions at the nanoscale, preferably with edges between 3 and 100 nm and the inside of which is hollow.

The term "tetrahedron" refers to the assembly of polypeptide chain that apparently forms the shape of a tetrahedron with dimensions at the nanoscale, preferably with edges between 3 nm and 100 nm and the inside of which is hollow.

The term "polypeptide chain" has a general meaning and refers to a sequence of amino acids that are connected by peptide bond between each contributing amino acids.

The term "segment" has a general meaning and refers to an amino acid sequence of the polypeptide chain that forms a defined homogeneous structural and design unit.

### Coiled-coil segment and coiled-coil-forming segment

The term "coiled-coil segment" has a general meaning and refers to a structural polypeptide motif comprising two or more helices that twist around each other forming an unperturbed super coil.

The term "coiled-coil-forming segment" denotes a segment on a single polypeptide chain that has the potential to form a coiled-coil segment in combination with one or more specific coiled-coil-forming segments.

Coiled-coil-forming segments usually contain a heptad repeat which can be designated (a-b-c-d-e-f-g)ₙ and which repeats approximately every two turns of a coiled-coil helix and in which each position represents an amino acid residue. Positions "a" and "d" are usually occupied by nonpolar, hydrophobic amino acid residues that are found at the interface of the two helices of the coiled-coil segments, "e" and "g" are solvent exposed and usually polar residues that interact electrostatically, while "b", "c" and "f" are usually hydrophilic residues and exposed to the solvent. Different amino acids at positions "a-g" of coiled-coil-forming segments define the oligomerization state, specify helix orientation and stability of coiled-coil segments. The specific amino acids present in each heptad may vary between each heptad of the sequence. More specifically, the term coiled-coil-forming segment in the description refers to naturally occurring or designed coiled-coil polypeptide structure motifs which comprise typically between 3 and 50 heptads and can form parallel or antiparallel, homo- or hetero- oligomers.

The coiled-coil-forming segments can be selected from the naturally occurring or designed coiled-coil protein or polypeptide structure motifs, preferentially from naturally occurring leucine-zipper proteins or from designed coiled-coil-forming sequences, more preferentially from SEQ ID NOs: 2, 4, 6, 8, 10, 12, 14, 16 and 18.

The orientation of chains (coiled-coil-forming segments) in the coiled-coil (dimer of coiled-coil-forming segments) can be either parallel (all protein chains run in the same direction) or antiparallel (chains run in opposite directions). Principles determining the identification and properties of coiled-coil-forming segments are well known to the researchers skilled in this subject, also the stability of parallel coiled-coil segments can be predicted from the sequence of existing polypeptides or designed as synthetic peptides (Hagemann UB, Mason JM, Müller KM, Arndt KM., J.Mol.Biol. 381(1):73-88, 2008).

The term "homodimer" in the description of the invention has a general meaning and refers to a complex composed of two monomeric segment of the same type.

The term "heterodimer" in the description of the invention has a general meaning and refers to a complex composed of two monomeric segments of different type.

The main characteristic of coiled-coil-forming segments to be selected for the inclusion into the same polypeptide chain is that each coiled-coil-forming segment interacts only or with high preference with its designated partner segment which was designed, and not with any other segment in the reaction mixture from the same polypeptide. For example, a homodimeric coiled-coil-forming segment should form a coiled-coil segment only with another identical coiled-coil-forming segment while a heterodimeric parallel coiled-coil-forming segment should interact only with the corresponding pairing segment, i.e. another defined heterodimeric coiled-coil-forming segment.

### Design of a single polyhedra-forming Polypeptide chain

A single polyhedra-forming polypeptide chain can be designed by linking together a defined number of coiled-coil-forming segments in defined order connected with selected linkers. An additional amino acid sequence can be added, preferentially located at N- or C- terminal ends.

The term "linker" refers to shorter amino acid sequences, whose role could be only to separate the individual domains or segments of the protein. Invention comprises the existence of nonhelical linker segments between coiled-coil regions that break the helix and provide the flexibility for the assembly between different peptide segments. Linker segments preferentially span between 1 and 10 amino acid residues and further preferentially include hydrophilic residues that break alpha-helix. Preferential residues for the linker are glycine, serine, threonine, proline, cysteine, histidine, tryptophan, tyrosine. A linker used in an example of a design of polypeptide chain for tetrahedron assembly of the invention is composed of the tetrapeptide Ser-Gly-Pro-Gly.

In contrast to fusion proteins disclosed in U.S. Patent No. 6,756,039 where protein domains are rigidly-linked by a rigid linker such as an alpha helix, a polypeptide of the present invention contains flexibly-linked polypeptide domains that function as a molecular hinge allowing free degree of rotation of the rigid coiled-coil segments.

The term "additional amino acid sequence" or "functional polypeptide" refers to an amino acid sequence that can be appended to the polypeptide at either the end of the polyhedron-forming polypeptide or between the coiled-coil-forming segments. Such an additional amino acid sequence is not necessary for the formation of polypeptide material into a polyhedron by self-assembly but endows the material with additional function, such as binding of metal ions, binding of fluorescent tags, intrinsic fluorescence, catalytic activity, stimulation of the immune response or binding of other molecules, such as other proteins, nucleic acids, polysaccharides, lipids or drugs.

The term "tag sequence" or "tag" refers to a sequence of amino acids, which is added to the polypeptide to facilitate purification/isolation/detection of the polypeptide. The position of tag sequence is optional but it must allow functional expression of the polypeptide and maintain the function for which these amino acid sequences were selected, which is known to experts in the field.

The term "DNA" in the description refers to a sequence of nucleotides having an open reading frame that encodes a polypeptide of the present invention, usually being operatively linked to regulatory elements, promoter and terminator to promote expression of the polypeptide in the host cells. The length of the DNA sequence may vary greatly depending on the particular polypeptide.

### Design of a polypeptide tetrahedron from a single polypeptide chain

One can treat the coiled-coil-forming segments as rigid rods that can be further linked to other coiled-coil-forming segments. By introducing a flexible linker between these segments, different orientations of the consecutive coiled-coil segments will be allowed. Coiled-coil-forming segments linked into a single polypeptide chain can be in principle used to form any geometric objects that can be split into polyhedra. Up to now mostly simple one-dimensional objects, such as fibrils or rods have been created from coiled-coil segments, where the polypeptide units contain coiled-coil segments that allow staggered packing mediated by the complementary coiled-coil segments (Woolfson DN, Pept. Science. 94(1): 118-127, 2010). Previously, the inventors have proposed the assembly of two- and three-dimensional objects by the introduction of polypeptide units that contain three coiled-coil-forming segments that allow formation of lattice or a cube; the last comprises six polypeptide units that form the twelve edges of the cube (Jerala R. et al., WO 2011/046521 A1). However, the assembly of building blocks into a geometric shape from several units may lead to heterogeneous products. Moreover, if different building blocks are used their precise concentration and precise stoichiometric ratios need to be adjusted in narrow ranges. It would be highly desirable to be able to construct a geometric object more easily, where the assembly does not depend on the precise concentration of each of its building elements similar as shown before for the DNA origami (Rothemund PWK., Nature. 440: 297-302, 2006). Such a constitution can be achieved using a single polypeptide chain in accordance with the present invention.

The inventors found out that for the stability of polyhedra constructed from a single polymer chain the path along the edges of a polyhedron has to obey certain conditions. Each edge of the polyhedron has to be crossed exactly twice. The forbidden paths are the following: (a) a path must not immediately turn around after visiting a vertex of the polyhedron and form an antiparallel dimer with its immediately preceding segment (Figure 1 A); (b) in each vertex of the polyhedron, a path that enters and exits the vertex must not form a dimer in the same or opposite direction connecting the same three edges (Figure 1B,C).

Based on those rules one can design paths that form different polyhedra of different complexities, such as shown on the examples of trigonal prism (Figure 2A), tetragonal prism (Figure 2B) or tetragonal pyramid (Figure 2C). In each of those cases each edge is formed of single coiled-coil dimer either in parallel or antiparallel orientation. A trigonal prism (Figure 2A) is composed of 18 coiled-coil forming segments that form 9 edges, a tetragonal prism, such as a cube, (Figure 2B) is formed of 24 coiled-coil-forming segments that form 12 edges, and a tetragonal pyramid (Figure 2C) requires 16 coiled-coil-forming segments that form its 8 edges. The simplest tree-dimensional polyhedral geometric body is a tetrahedron, which comprises six edges connecting three edges at each of its four vertices. The inventors invented a procedure to design and prepare a stable tetrahedron from a single polypeptide chain so that the polypeptide chain by traversing all of its edges in a single connected path so that each edge is crossed exactly twice.

The inventors analysed the possible arrangements of the polypeptide path along the edges of a tetrahedron and concluded that it is not possible to construct a tetrahedron from a single chain, which comprises only antiparallel units. It is also not possible to construct a tetrahedron from a single chain which peptide segments form only parallel dimers that represent tetrahedron edges. The only possible topologies for the formation of a tetrahedron combine both antiparallel and parallel pairs at the edges of the polypeptide tetrahedron. The solutions of this path are the following possibilities which have been found by the present inventors: (a) a tetrahedron whose edges are composed of four parallel and two antiparallel edges and (b) a tetrahedron whose edges are composed of three parallel and three antiparallel dimeric coiled-coil segments (Figure 1G).

The analysis of those invented solutions also shows that the beginning and end of the polypeptide path along the edges of a tetrahedron coincide at the same vertex. This means that the additional amino acid sequences at both N- and C-terminal ends coincide at the same vertex. Therefore this property can be used for the reconstitution of a selected protein that is split into two segments which reassemble when the polyhedron assembles. This has several useful applications such as the detection for the polyhedral assembly by activation of an enzymatic activity (triggering the enzyme activity) or reconstitution of a fluorescent protein. Moreover, the addition of a selected polypeptide can endow a functional assembly with additional technologically useful properties such as enzymatic activity, light fluorescence or absorbance, activation of immune response, activation of cell signalling, cell binding, trafficking, cell penetration, biomineralization, deposition of metals, clusters of charges of hydrophobic residues or different arrangements of aminoacid residues as used in the natural protein structures.

For the formation of a tetrahedron the order of parallel and antiparallel coiled-coil dimer-forming units has to be precisely selected so that the correct orientation of defined coiled-coil-forming segment pairs, parallel or antiparallel, is achieved on each edge and correct connection between the edges that leads to the formation of the desired polyhedral topology is achieved. In the example of tetrahedral self-assembly the following nine coiled-coil-forming segments that have been selected from the designed and natural coiled-coil-forming segments: SEQ ID NOs: 2, 4, 6, 8, 10, 12, 14, 16, 18. Many other polypeptide sequences could be selected by the person skilled in understanding the principles of the selectivity of the coiled-coil-forming segments.

Since the beginning and end of the polypeptide chain along the edges of the tetrahedron coincide, the same tetrahedron could be assembled using the polypeptide chain with the circular permutations of the selected sequence. Circular permutation denotes removing one or several coiled-coil-forming segments from one end and attaching them in the same order to the other end of the polypeptide chain.

### Recombinant nucleic acid

Standard methods of molecular biology can be used in the invention and are generally known to experts in the field (Sambrook et al. 1989. Molecular Cloning: A laboratory manual, 2nd ed., Cold Spring Harbor, NY, Ausubel et al. Current Protocols in Molecular Biology, Green Publishing Associates, Inc. and John Wiley & Sons, Inc., NY).

The invented polypeptide material can be synthesized by expressing DNA coding for the polypeptide in a suitable host organism. The DNA coding for the polypeptide is inserted in an appropriate expression vector. Suitable vectors include, but are not limited to: plasmids, viral vectors, etc. Expression vectors, which are compatible with the host organism cells, are well known to experts in the field and include the appropriate control elements for transcription and translation of nucleic acid sequence. Typically, an expression vector includes an expression cassette, which includes in 5' to 3' direction the promoter, the coding sequence for the fusion protein operatively linked with the regulatory elements, promoter and terminator, including a stop codon for RNA polymerase and polyadenylation signal for polyadenylase.

The expression vector may be prepared for expression in prokaryotic and eukaryotic cells. For example, prokaryotic cells are bacteria, primarily *Escherichia coli.* According to the invention, prokaryotic cells are preferably used to obtain sufficient quantity of the polypeptide. The expression vector usually contains the operationally associated control elements which are operationally linked to the DNA of the invention, which codes for the protein. The control elements are selected in a way to trigger efficient and tissue-specific expression. The promoter may be constitutive or inducible, depending on the desired pattern of expression. The promoter may be of native or foreign origin (not represented in the cells, where it is used), and may be natural or synthetic. The promoter must be chosen in order to work in the target cells of the host organism. In addition, initiation signals for the efficient translation of fusion protein are included, which comprises the ATG sequence and the corresponding sequences. When the vector, used in the invention, includes two or more reading frames, the reading frames should be operationally associated with control elements independently and the control elements should be the same or different, depending on the desired production of proteins.

Examples of bacterial expression vectors include, but are not limited to: pET vectors, pRSET vectors, and others. When vectors are used in the bacterial cells, the control elements are of bacterial origin.

Examples of mammalian expression vectors for mammalian cells include, but are not limited to: pcDNA (Invitrogen), pFLAG (Sigma), and others. When vectors are used in mammalian cells, the control elements are in most cases of viral origin, for example, adenovirus 2, cytomegalovirus, a virus Simian virus 40.

### Process for the preparation of polypeptide material for the self-assembly into polyhedra

The disclosure made specifically refers to a process for generating the polypeptide material of the invention and includes: introduction of polypeptide-coding DNA into a host organism, cultivation of host organism under conditions suitable for polypeptide expression, polypeptide isolation and purification with commonly used biochemical purification techniques including chromatography.

According to the disclosure, a fusion polypeptide can be synthesized in the host organism that expresses the heterologous nucleic acid, which encodes the fusion polypeptide based on the universal genetic code. The fusion polypeptide of the invention may be used for the preparation of polypeptide material.

In general, the heterologous nucleic acid is incorporated into an expression vector (viral or non-viral), which is described above.

The disclosure includes host cells and organisms that contain nucleic acid according to the invention (transient or stable), which codes for the fusion polypeptide according to the invention. Appropriate host organisms are known to the experts in the field of molecular biology and include bacterial and eukaryotic cells.

The transfer of vectors into host cells can be carried out by conventional methods known in the art and described above.

The DNA transfer may be transient or stable. Transient expression refers to the introduction of the vector DNA, which according to the disclosure is not incorporated into the genome of cells. Stable intake is achieved by incorporating DNA of the invention into the host genome. The transfer of the DNA according to the invention, especially for the preparation of the host organism, which has a stable DNA integrated, may according to the disclosure be controlled by the presence of markers. DNA coding for markers refers to resistance to drugs, for example antibiotics, and may be included in the vector with the DNA according to the invention, or on a separate vector.

### Self-assembly of the tetrahedron

The assembling of tetrahedron can be achieved by exposition of solubilized denatured polypeptide material to specific conditions inducing the nanostructure formation. Well designed nanostructures represent the most stable self-assembly of the given polypeptide sequence, which is more stable than other alternative assemblies. For the assembly of a large number of components the energetic difference between the most stable target structure and others becomes relatively smaller and the system requires longer time to achieve the most stable arrangement. This can be accomplished by keeping the system under the conditions of a dynamic exchange between unfolded and assembled components. Inventors found a procedure to improve the assembly of polypeptide nanostructures that allows sufficient time for the assembly of the most stable structures by a very slow decrease of the concentration of denaturing agent, where the polypeptide that forms the nanostructures is dialyzed from the conditions where it is completely unfolded and disassembled against a slowly decreasing concentration of a dialyzing solution by slowly adding buffer into the dialysis solution under mixing. The refolding and nanostructure assembly thus occurs over a period of preferably more than 16 hours, more preferably between 16 and 40 hours. Range of the starting and ending concentration of the chemical denaturant in the dialyzing solution can be determined from the spectroscopic analysis of the stability of the tertiary and secondary structure of the investigated polypeptide chain in the presence of different concentrations of denaturing agent or by the investigation of the stability of all the coiled-coil dimers that are concatenated in the polyhedral polypeptide. Different denaturing agents can be used for this procedure, such as guanidinium hydrochloride, urea, LiBr or other chemical agents that disrupt the secondary structure of coiled-coil segments at high concentrations, typically 4-5 M guanidinium hydrochloride whereas the nanostructure assembly occurs at lower concentrations, typically less than 1 M guanidinium hydrochloride.

Examples of implementation, designed to illustrate the invention, are shown below. The descriptions of examples of implementation have no intention of limiting the invention and should be understood as a demonstration of the invention. Further, these may be changed without deviating from the scope of the invention.

### Examples

### Example 1: Selection of coiled-coil-forming segments

The most important interactions governing the assembly of coiled-coil segments are well known to the experts in the field. Formation of stable nanostructures according to this invention requires that the coiled-coil forming segments used in the design are orthogonal, meaning that individual coiled-coil-forming segment can form coiled-coil dimer simultaneously exclusively with its designated pair partner and not with other coiled-coil-forming segments present in the reaction mixture. Pairs of coiled-coil-forming segments were selected among natural (BCR, Taylor CM. and Keating AE., Biochem., 44:16246-16256, 2005; GCN, Harbury PB. et al., Science, 262(5138): 1401-1407, 1993) and *de novo* designed (APH, Gurnon DG. et al., JACS, 125:7518-7519, 2003; P3-P8, Jerala R. and Gradisǎr H., J. Pept. Science, 17:100-106, 2011) coiled-coils. Heterodimeric parallel coiled-coil-forming peptides (P3,P4,P5,P6,P7,P8), homodimeric parallel (GCNshort) and homodimeric antiparallel coiled-coil-forming peptides (APH, BCR) from Table 1 were used in the design of a tetrahedron-forming polypeptide chain.

**Table 1: Sequences of coiled-coil-forming segments used in designed polypeptide. Antiparallel homodimer-forming peptide BCR and parallel homodimer-forming peptide GCNshort (derived from GCN) occur in nature whereas antiparallel homodimer-forming peptide APH and parallel heterodimeric pairs P3-P4, P5-P6, P7-P8 are de novo designed based on the known principles of coiled-coil dimer formation.**

| | | |
|---|---|---|
| **APH** | MKQLEKELKQLEKELQAIEKQLAQLQWKAQARKKKLAQLKKKLQA | SEQ ID NO: 2 |
| **BCR** | DIEQELERAKASIRRLEQEVNQERSRMAYLQTLLAK | SEQ ID NO: 4 |
| **GCNshort** | QLEDKVEELLSKNYHLENEVARLKKLVG | SEQ ID NO: 6 |
| **P3** | SPEDKIAQLKEKNAALKEKNQQLKEKIQALKYG | SEQ ID NO: 8 |
| **P4** | SPEDKIAQLKQKIQALKQENQQLEEENAALEYG | SEQ ID NO: 10 |
| **P5** | SPEDENAALEEKIAQLKQKNAALKEEIQALEYG | SEQ ID NO: 12 |
| **P6** | SPEDKNAALKEEIQALEEENQALEEKIAQLKYG | SEQ ID NO: 14 |
| **P7** | SPEDEIQALEEKNAQLKQEIAALEEKNQALKYG | SEQ ID NO: 16 |
| **P8** | SPEDKIAQLKEENQQLEQKIQALKEENAALEYG | SEQ ID NO: 18 |

### Example 2: Design of the polypeptide chain composed of twelve coiled-coil-forming segments

inventors found that only two of the possible combinations of twelve coiled-coil-forming segments within a single polypeptide chain can lead to the assembly of a tetrahedron, namely a combination of four parallel and two antiparallel coiled-coil dimers and a combination of three parallel and three antiparallel coiled-coil segments. Inventors designed an example of a polypeptide chain comprising the following combination of twelve coiled-coil-forming segments (Figure 3A) that can self-assemble into a tetrahedron (Figure 3B):
(1) antiparallel homodimer-forming segment APH - (2) parallel heterodimer-forming segment P3 - (3) antiparallel homodimer-forming segment BCR - (4) parallel homodimer-forming segment GCNsort - (5) antiparallel homodimer-forming segment APH - (6) parallel heterodimer-forming segment P7 - (7) parallel homodimer-forming segment GCNshort - (8) parallel heterodimer-forming segment P4 - (9) parallel heterodimer-forming segment P5 - (10) parallel heterodimer-forming segment P8 - (11) antiparallel homodimer-forming segment BCR - (12) parallel heterodimer-forming segment P6
where a pair of antiparallel homodimer-forming segments APH and BCR form antiparallel homodimeric coiled-coils (APH-APH, BCR-BCR), and parallel homodimer-forming segment GCNshort forms a parallel homodimeric coiled-coil (GCNshort-GCNshort). The segments APH, BCR, GCNshort form dimers exclusivelly with an identical segment, but not with other types of homodimer-forming segments; and where parallel heterodimer-forming segments P3, P5, P7 form parallel heterodimeric coiled-coils exclusively with their partners P4, P6, P8, respectively. This example is a case of a design of four parallel and two antiparallel coiled-coil segments that form six edges of a tetrahedron.

To obtain a tetrahedron, the domains have to be arranged in a defined order. All domains of the polypeptide chain are coiled-coil-forming segments and each of them forms a coiled-coil only with its partner peptide from the same polypeptide chain. Coiled-coil-forming segments were selected among the sequences specified in the Table 1: SEQ ID NO: 2,4,6,8,10,12,14,16,18.

For the formation of a tetrahedron or any other polyhedra by self-assembly, the linkers between the coiled-coil-forming segments need to have the freedom to freely rotate in order to assemble into the designed arrangement (i.e.. flexible linkers). For this purpose the inventors introduced between all coiled-coil-forming segments a polypeptide linker that has to perform several tasks: it has to prevent formation of a continuous helix from two consecutive coiled-coil-forming segments, it has to allow free rotation and it has to allow crossing of two or more linkers between polypeptide chains at each vertex. For this purpose the inventors selected the tetrapeptide Ser-Gly-Pro-Gly (SGPG), which contains three strong helix breaking residues (glycine and proline) that are flexible, small and hydrophilic.

Additionally a hexahistidine peptide tag was added to the N-terminal end of the polypeptide chain in order to facilitate purification and detection of the product.

The additional amino acid segments can be added at N- and/or C-terminal ends. The split yellow fluorescent protein (YFP) was added, NYFP at N-terminus and CYFP at C-terminus (Figure 10A). Simultaneously with the tetrahedron assembly, the protein YFP was reconstituted (Figure 10B) and its fluorescence was detected. In case of irregular polypeptide assembly the fluorescence measurement would have failed.

### Example 3_{.} Design and preparation of DNA constructs

The amino acid sequence of a complete polypeptide chain comprising all twelve coiled-coil-forming segments, eleven linker tetra-peptides between each two coiled-coil-forming segments, a hexahistidine peptide tag, and optionally additional amino acid sequence for yellow fluorescent protein (YFP) was reverse translated into the nucleotide sequence using the preferred codon usage for bacteria, where it was produced in a recombinant form.

A person skilled in the art could use other production organisms such as yeast, mammalian cells or other eukaryotic cells for the production of a polypeptide coding for a tetrahedron, where the codon usage and appropriate vectors and cloning procedures would be modified accordingly.

DNA sequences coding for peptide domains described above were designed from the amino acid sequences using tool Gene Designer from DNA2.0 Inc. (http://www.biocompare.com/ProductDetails/505315/Gene-Designer.html) that enables the user to design DNA fragments and optimize expression for the desired hosts (e.g. *E.coli*) using codon optimization. Gene composed of all DNA fragment sequences in regular order was obtained using standard procedures, cut out from the vector with restriction endonucleases and cloned into the appropriate vector containing required regulatory sequences known to the experts in the field. Commercial vector pSB1A2 (http://partsregistry.org/Part:pSB1A2) carrying all necessary features such as antibiotic resistance, origin of replication and multiple cloning site, was used as vector.

The inventors prepared the DNA construct (SEQ ID NO: 19) coding for the polypeptide chain (SEQ ID NO: 20) composed of the following twelve coiled-coil-forming segments in this order: APH-P3-BCR-GCNshort-APH-P7-GCNshort-P4-P5-P8-BCR-P6. Additionally, the inventors prepared the DNA construct (SEQ ID NO: 21) coding for the same polypeptide with split yellow fluorescence protein (YFP) segments added at both N- and C-terminal ends (SEQ ID NO: 22).

Common molecular biology methods (DNA fragmentation with restriction endonucleases, DNA amplification using polymerase chain reaction-PCR, DNA concentration detection, agarose gel electrophoresis, purification of DNA fragments from agarose gels, ligation of DNA fragments into a vector, transformation of chemically competent cells *E.coli* DH5α, isolation of plasmid DNA with commercially available kits, screening and selection) were used for preparation of DNA constructs. All procedures were performed under sterile conditions. DNA segments were characterized by restriction analysis and sequencing.

Molecular cloning procedures are well known to the experts in the field and are described, for example, in details in molecular biology handbook (Sambrook J., Fritsch E.F., Maniatis T. 1989. Molecular cloning: A laboratory manual. 2nd ed. New York, Cold Spring Harbor Laboratory Press: 1659 p.).

### Example 4: Preparation of recombinant tetrahedron-forming polypeptide SEQ ID NO 20

DNA construct (SEQ ID NOs: 19) coding for a tetrahedron-forming polypeptide (SEQ ID NO: 20) was prepared to demonstrate the feasibility for polypeptide assembly into tetrahedron. Both polypeptide chains consist of four antiparallel homodimerization domains, two parallel homodimerization domains and six parallel heterodimerization domains that self-assemble into six edges of a tetrahedron.

Plasmids encoding open reading frames of fusion proteins from Figures 3A, 10A were transformed into chemically competent *E. coli* BL21 (DE3) pLysS cells. Selected bacterial colonies grown on LB plates supplemented with selected antibiotic ampicillin were inoculated into 100 mL of LB growth media supplemented with antibiotic and grown overnight at 37°C and 160 rpm. Next day the overnight cultures were diluted 20-50-times reaching OD₆₀₀ of diluted cultures between 0.1 and 0.2. Then the bacterial cultures were grown until OD₆₀₀ reached 0.7 and polypeptide expression was induced by the addition of 1 mM inducer IPTG. Four hours after induction culture broths were centrifuged at 5000 rpm followed by resuspension of bacterial cells in lysis buffer (Tris pH 8.0, 0.1% deoxycholate supplemented with protease inhibitor cocktail) and frozen at -80 °C. Thawed cell suspensions were further lysed by sonication and centrifuged. Precipitate (residual non-lysed cells, inclusion bodies) and supernatant were checked for expression of polypeptides by SDS-PAGE (Figure 4) and when necessary by Western blot using anti-His-tag antibodies as primary antibodies. Produced recombinant polypeptides were mainly present in the insoluble part (inclusion bodies), which was composed of >80 % of the overexpressed polypeptide. Inclusion bodies were dissolved in 6 M GdnHCl (pH 8.0) and loaded on Ni²⁺-NTA column (Quiagen, GE). Purification under denaturing conditions was followed according to the manufacturer's instructions. After elution with 250 mM imidazole (pH 5.8) fractions containing polypeptides were combined and dialyzed twice against MiliQ water. Fractions containing polypeptides were analyzed by SDS-PAGE and Western blot. Degradation products were detected after purification by affinity chromatography, therefore further purification with reverse-phase liquid chromatography (HPLC-RP) was employed to obtain > 90 % purity of polypeptide which was confirmed by SEC (Figure 5).

### Example 5: Preparation of recombinant tetrahedron-forming polypeptide SEQ ID NO 22

Example 4 was repeated using DNA construct (SEQ ID NOs: 21) coding for the tetrahedron-forming polypeptide fused with a split-YFP protein (SEQ ID NO: 22). Precipitate (residual non-lysed cells, inclusion bodies) and supernatant were checked for expression of polypeptides by SDS-PAGE. The results are shown in Figure 11.

### Example 6: Refolding of polypeptide SEQ ID NO: 20

Purified polypeptide SEQ ID NO: 20 from Example 4 in 6 M guanidinium hydrochloride was diluted to low concentration of 5 µ/ml in 6 M guanidinium hydrochloride in 20 mM HEPES buffer pH 8.5, 150 mM NaCl. 50 ml of the solution was placed in a dialysis bag with membrane cutoff of 3.5 kDa, and placed in 20 mM HEPES buffer (pH 8.5) and 150 mM NaCl. Solution was stirred at 4 °C and 500 rpm for 20 h. After dialysis, the sample was concentrated with 30 kDa molecular weight cut-off concentrator following the flow-through with a 100 kDa molecular weight cut-off concentrator to remove the larger aggregates.

### Example 7: Controlled refolding of polypeptide SEQ ID NO: 20

Similar to Example 6, purified polypeptide SEQ ID NO: 20 from Example 4 in 6 M guanidinium hydrochloride was diluted to a concentration of 5 µ/ml in the same 6 M GdnHCl buffer. Solution with protein sample was inserted into a dialysis bag with membrane cutoff of 3.5 kDa and placed into a solution of 6 M GdnHCl. Solution of buffer of 20 mM HEPES buffer (pH 8.5) without GdnHCl was slowly added by a programmable pump to the dialysis solution, thereby slowly decreasing the concentration of GdnHCl to 0.5 M over a period of 20 hours.

### Example 8: Refolding of polypeptides SEQ ID NO: 22

Purified polypeptide SEQ ID NO: 22 from Example 5 in 6 M guanidinium hydrochloride was diluted to 2 mg/ml in 6 M guanidinium hydrochloride in 50 mM Tris buffer (pH 8.0), 300mM NaCl, 5 mM MgCl₂, 2 mM β-mercaptoethanol. 50 ml of the solution was placed in a dialysis bag with membrane cutoff of 3.5 kDa, and placed in 50 mM Tris buffer (pH 8.0), 300mM NaCl, 5 mM MgCl₂, 2 mM β-mercaptoethanol. Solution was stirred at 4 °C and 500 rpm for 20 h. After dialysis, the sample was concentrated with 30 kDa molecular weight cut-off concentrator following the flow-through with a 100 kDa molecular weight cut-off concentrator to remove the larger aggregates.

### Example 9: Determination of the polypeptide SEQ ID NO: 20 characteristics

The presence of a large fraction of α-helical secondary structure in the self-assembled tetrahedral polypeptide was confirmed by measuring the circular dichroism (CD) spectrum of the purified polypeptide SEQ ID NO: 20 solution in the range of 260 and 200 nm on a CD spectrometer Applied Photophysics (Figure 6A).

Purified polypeptide SEQ ID NO: 20 dissolved in 6 M GdnHCl was diluted to the final concentration of 0.1 mg/ml in 20 mM HEPES buffer solutions pH 8.5, containing different concentrations of GdnHCl, in the range from 6 M to a solution without denaturing agent. Secondary structure of the polypeptide at different concentrations of GdnHCl was determined by measuring the CD spectra (Figure 6B).

Thermal unfolding (Figure 6C) and subsequent refolding (Figure 6D) curves for polypeptide were recorded without denaturant in solution by measuring circular dichroism at 222 nm through every 1 °C/min ramp. The thermal unfolding was reversible.

The size of polypeptide SEQ ID NO: 20 in denaturant solution (6 M GdnHCl, Figure 7A) and after refolding of polypeptide (0 M GdnHCl, Figure 7B) were determined on ZetasizerNano (Malvern, UK). It was confirmed that a hydrodynamic diameter (H_{d}) of denatured molecules (9.45 nm) is larger than H_{d} of refolded structure (6.88 nm) which is in accordance with computer designed structure.

Sample with the resulting self-assembled nanostructure was analyzed by transmission electron microscope (TEM). Sample obtained after refolding of polypeptide SEQ ID NO: 20 was deposited on the grid for TEM analysis and stained with uranyl acetate. Using a different sample, staining with uranyl acetate was performed after incubation of Nanogold 2 nm reagent (Nanoprobes, Yaphank, NY, USA) with sample on the grids. Images from TEM demonstrated formation of tetrahedrons with edges from 5 nm to 10 nm (Figure 8). When Nanogold reagent was employed, 2 nm black dots were observed in one of the vertices of the tetrahedron, which is in accordance with the reagent binding to the hexahistidine tag of the polypeptide.

Sample with the resulting self-assembled nanostructure tetrahedron was analyzed by atomic force microscope (AFM), which demonstrated the presence of small nanoparticles, measuring less than 10 nm (Figure 9), which was in accordance with the predictions of the self-assembled tetrahedron with edges composed of coiled-coils.

### Example 10: Determination of the polypeptide SEQ ID NO: 22 characteristics

The correct self-assembly of the polypeptide SEQ ID NO: 22 was verified by measuring the intrinsic fluorescence of the reconstituted YFP from split segments genetically linked to the N- and C-terminal segment of the tetrahedral polypeptide. The solution exhibited distinctive fluorescence (Figure 12) which indicated that the beginning and end of the tetrahedral polypeptide converge at the same vertex in a correctly folded nanostructure.

Sample with the resulting assembled nanostructure was analyzed by TEM. Sample obtained after refolding of polypeptide SEQ ID NO: 22 was deposited on the grid for TEM analysis and staining with uranyl acetate was performed after incubation of Nanogold 2 nm reagent with sample on the grid. Images from TEM demonstrated formation of tetrahedrons with edges from 5 nm to 10 nm (Figure 13). 2 nm in size black dots were observed in one of the vertices of the tetrahedron, which is in accordance with reagent binding to the hexahistidine tag on the polypeptide.

Sample with the resulting assembled nanostructure tetrahedron was analyzed by AFM, which demonstrated the presence of small nanoparticles, measuring around 20 nm (Figure 14). The shapes of structures were in accordance with the prediction of the assembled tetrahedron with additional reconstituted YFP at one vertex.

### Technological and pharmaceutical applicability

Nano-tetrahedra made of polypeptides can be useful in many technological applications. They can be used to encase nanoparticles for different applications, such as drug delivery, for chemical catalysis within the polypeptide polyhedral nano-cage or at the surface of the assembled nanostructure, for binding of specific compounds, as a carrier of vaccines, antitumor or other pharmaceutical agents, assembly of inorganic or organic compounds at the defined positions of the polypeptide polyhedral scaffold, for diagnostics, based on the self-assembly or reconstitution of split protein domains inserted at selected positions within the polyhedral polypeptide structure.

## Claims

1. Polypeptide capable of forming a polyhedron by self-assembly, wherein the polypeptide contains at least 12 coiled-coil-forming segments connected via linker segments, wherein each of the edges of the polyhedron is constructed of a pair of said coiled-coil-forming segments and each pair of said coiled-coil-forming segments forms a coiled-coil structure.

2. Polypeptide according to claim 1, wherein each coiled-coil-forming segment of a pair of coiled-coil-forming segments has higher probability of forming a coiled-coil structure with the other coiled-coil-forming segment of said pair of coiled-coil-forming segments than with any other coiled-coil-forming segment of the polypeptide.

3. Polypeptide according to claim 1 or 2, wherein the nonhelical linker segments each consist of at least 1 amino acid residues, preferably 1 to 10 amino acid residues, more preferably 2 to 6 amino acid residues, most preferably 4 amino acid residues.

4. Polypeptide according to one of claims 1 to 3, wherein the nonhelical linker segments are tetrapeptide segments with the amino acid sequence SGPG.

5. Polypeptide according to one of claims 1 to 4, wherein the polypeptide contains at least one of the coiled-coil-forming segments represented by SEQ ID NOs. 2, 4, 6, 8, 10, 12, 14, 16 and 18.

6. Polypeptide according to one of claims 1 to 5, wherein the polypeptide contains at least one coiled-coil-forming segment derived from naturally occurring leucine-zipper proteins.

7. Polypeptide according to one of claims 1 to 6, wherein the polyhedron is a tetrahedron.

8. Polypeptide according to claim 7, wherein the sequential order of coiled-coil-forming segments is Aa-Pa-Ab-Pb-Aa'-Pc-Pb'-Pa'-Pd-Pc'-Ab'-Pd' or a cyclic permutation thereof, wherein Aa and Ab represent coiled-coil-forming segments forming antiparallel coiled-coils with Aa' and Ab', respectively, and Pa to Pd represent coiled-coil-forming segments forming parallel coiled-coils with Pa' to Pd', respectively.

9. Polypeptide according to claim 7 or 8, wherein the sequential order of coiled-coil-forming segments is APH-P3-BCR-GCN-APH-P7-GCN-P4-P5-P8-BCR-P6 represented by SEQ ID NOs 2, 8, 4, 6, 2, 16, 6, 10, 12, 18, 4, and 14, respectively, or a cyclic permutation thereof.

10. Polypeptide according to claim 7, wherein three out of six edges of the tetrahedron are composed of antiparallel coiled-coil dimers and three of parallel coiled-coil dimers.

11. Fusion polypeptide of a polyhedron-forming polypeptide according to one of claims 1 to 10 with a functional polypeptide domain attached to the N- and/or C-terminal segment of the polyhedron-forming polypeptide.

12. Fusion polypeptide according to claim 11, wherein the functional polypeptide domain is split into two segments that are linked to the N-terminus and C-terminus of the polypeptide, respectively, wherein the two segments of the functional polypeptide domain reassemble upon formation of a tetrahedron and gain function.

13. Polypeptide polyhedron formed from a polypeptide according to one of claims 1 to 10 or a fusion polypeptide according to one of claims 11 to 12.

14. Polypeptide polyhedron, whose edges are composed of linked coiled-coil-forming dimeric segments, wherein the number of said segments is twice the number of the edges of the polyhedron, wherein the polypeptide forms a closed path along the edges of the polyhedron and nonhelical linker segments are located between the coiled-coil-forming dimeric segments and form the vertices of the polyhedron.

15. DNA coding for a polypeptide according to one of claims 1 to 10, a fusion polypeptide according to one of claims 11 to 12, or a polypeptide polyhedron according to claim 13 or 14.

16. A drug encaged into a polyhedral nanocage formed from a polypeptide according to one of claims 1 to 10 or fusion polypeptide according to one of claims 11 to 12 for use in the treatment of or for use in diagnosing a human or animal.

17. The drug encaged into a polyhedral nanocage formed from a polypeptide according to one of claims 1 to 10 or fusion polypeptide according to one of claims 11 to 12 for use according to claim 16, wherein the polypeptide contains target-specific binding substances directing the polyhedral nanocage to a desired tissue.

## Patentansprüche

1. Polypeptid, das durch Selbstassemblierung ein Polyeder bilden kann, wobei das Polypeptid mindestens 12 über Linker-Segmente verbundene, Coiled-Coil-bildende Segmente enthält, wobei jede der Kanten des Polyeders aus einem Paar der Coiled-Coil-bildenden Segmente aufgebaut ist und jedes Paar der Coiled-Coil-bildenden Segmente eine Coiled-Coil-Struktur bildet.

2. Polypeptid nach Anspruch 1, wobei jedes Coiled-Coil-bildende Segment von einem Paar von Coiled-Coil-bildenden Segmenten eine höhere Wahrscheinlichkeit zur Bildung einer Coiled-Coil-Struktur mit dem anderen Coiled-Coil-bildenden Segment des Paars von Coiled-Coil-bildenden Segmenten aufweist, als mit jedem anderen Coiled-Coil-bildenden Segment des Polypeptids.

3. Polypeptid nach Anspruch 1 oder 2, wobei die nicht-helikalen Linker-Segmente jeweils aus mindestens 1 Aminosäurerest bestehen, vorzugsweise 1 bis 10 Aminosäureresten, besonders bevorzugt 2 bis 6 Aminosäureresten, am meisten bevorzugt 4 Aminosäureresten.

4. Polypeptid nach einem der Ansprüche 1 bis 3, wobei die nicht-helikalen Linker-Segmente Tetrapeptidsegmente mit der Aminosäuresequenz SGPG sind.

5. Polypeptid nach einem der Ansprüche 1 bis 4, wobei das Polypeptid mindestens eines der Coiled-Coil-bildenden Segmente enthält, die durch die SEQ ID Nr. 2, 4, 6, 8, 10, 12, 14, 16 und 18 dargestellt sind.

6. Polypeptid nach einem der Ansprüche 1 bis 5, wobei das Polypeptid mindestens ein aus natürlich vorkommenden Leucin-Zipper-Proteinen abgeleitetes, Coiled-Coilbildendes Segment enthält.

7. Polypeptid nach einem der Ansprüche 1 bis 6, wobei das Polyeder ein Tetraeder ist.

8. Polypeptid nach Anspruch 7, wobei die sequenzielle Reihenfolge der Coiled-Coil-bildenden Segmente Aa-Pa-Ab-Pb-Aa'-Pc-Pb'-Pa'-Pd-Pc'-Ab'-Pd' ist oder eine zyklische Permutation davon, wobei Aa und Ab Coiled-Coil-bildende Segmente darstellen, die antiparallele Coiled-Coils mit Aa' bzw. Ab' bilden, und Pa bis Pd Coiled-Coil-bildende Segmente darstellen, die parallele Coiled-Coils mit jeweils Pa' bis Pd' bilden.

9. Polypeptid nach Anspruch 7 oder 8, wobei die sequenzielle Reihenfolge der Coiled-Coil-bildenden Segmente APH-P3-BCR-GCN-APH-P7-GCN-P4-P5-P8-BCR-P6 ist, die durch SEQ ID Nr. 2, 8, 4, 6, 2, 16, 6, 10, 12, 18, 4 bzw. 14 dargestellt sind, oder eine zyklische Permutation davon.

10. Polypeptid nach Anspruch 7, wobei drei der sechs Kanten des Tetraeders aus antiparallelen Coiled-Coil-Dimeren und drei aus parallelen Coiled-Coil-Dimeren zusammengesetzt sind.

11. Fusionspolypeptid eines Polyederbildenden Polypeptids nach einem der Ansprüche 1 bis 10 mit einer an das N- und/oder C-terminale Segment des polyederbildenden Polypeptids gebundenen funktionellen Polypeptiddomäne.

12. Fusionspolypeptid nach Anspruch 11, wobei die funktionelle Polypeptiddomäne in zwei Segmente, die mit dem N-Terminus bzw. dem C-Terminus des Polypeptids verknüpft sind, gespalten sind, wobei die beiden Segmente der funktionellen Polypeptiddomäne sich bei der Bildung einer Tetraeder- und Verstärkungsfunktion erneut assemblieren.

13. Polypeptidpolyeder, gebildet aus einem Polypeptid nach einem der Ansprüche 1 bis 10 oder einem Fusionspolypeptid nach einem der Ansprüche 11 bis 12.

14. Polypeptid-Polyeder, dessen Kanten aus verketteten Coiled-Coil-bildenden dimeren Segmenten zusammengesetzt sind, wobei die Anzahl der Segmente doppelt so groß ist wie die Anzahl der Kanten des Polyeders, wobei das Polypeptid einen geschlossenen Pfad entlang der Kanten des Polyeders bildet und sich nicht-helikale Linker-Segmente zwischen den Coiled-Coil-bildenden dimeren Segmenten befinden und die Ecken des Polyeders bilden.

15. DNA, die für ein Polypeptid nach einem der Ansprüche 1 bis 10 kodiert, Fusionspolypeptid nach einem der Ansprüche 11 bis 12 oder Polypeptid-Polyeder nach Anspruch 13 oder 14.

16. In einen polyedrischen Nanokäfig eingekoppeltes Arzneimittel, gebildet aus einem Polypeptid nach einem der Ansprüche 1 bis 10 oder Fusionspolypeptid nach einem der Ansprüche 11 bis 12 zur Verwendung bei der Behandlung oder zur Anwendung bei der Diagnose von Mensch oder Tier.

17. In einen polyedrischen Nanokäfig eingekoppeltes Arzneimittel, gebildet aus einem Polypeptid nach einem der Ansprüche 1 bis 10 oder Fusionspolypeptid nach einem der Ansprüche 11 bis 12 zur Verwendung nach Anspruch 16, wobei das Polypeptid zielspezifische Bindungssubstanzen enthält, die den polyedrischen Nanokäfig auf das gewünschte Gewebe richten.

## Revendications

1. Polypeptide capable de former un polyèdre par auto-assemblage, le polypeptide contenant au moins 12 segments formant des superhélices reliées par l'intermédiaire de segments de liaison, chacun des côtés du polyèdre étant constitué d'une paire desdits segments formant des superhélices et chaque paire desdits segments formant des superhélices constituant une structure de superhélices.

2. Polypeptide selon la revendication 1, chaque segment formant des superhélices d'une paire de segments formant des superhélices ayant une probabilité supérieure de former une structure de superhélices avec l'autre segment formant des superhélices de ladite paire de segments formant des segments de superhélices qu'avec n'importe quel autre segment formant des superhélices du polypeptide.

3. Polypeptide selon la revendication 1 ou 2, dans lequel les segments de liaison non hélicoïdaux sont chacun constitués d'au moins 1 résidu d'acides aminés, de préférence de 1 à 10 résidus d'acides aminés, plus préférablement de 2 à 6 résidus d'acides aminés, le plus préférablement de 4 résidus d'acides aminés.

4. Polypeptide selon l'une des revendications 1 à 3, les segments de liaison non hélicoïdaux étant des segments tétrapeptidiques avec la séquence d'acides aminés SGPG.

5. Polypeptide selon l'une des revendications 1 à 4, le polypeptide contenant au moins l'un des segments formant des superhélices représentés par les SEQ ID N° 2, 4, 6, 8, 10, 12, 14, 16 et 18.

6. Polypeptide selon l'une des revendications 1 à 5, le polypeptide contenant au moins un segment formant des superhélices dérivé de protéines en fermeture éclair à leucines d'origine naturelle.

7. Polypeptide selon l'une des revendications 1 à 6 dans lequel le polyèdre est un tétraèdre.

8. Polypeptide selon la revendication 7 dans lequel l'ordre séquentiel des segments formant des superhélices est Aa-Pa-Ab-Pb-Aa'-Pc-Pb'-Pa'-Pd-Pc'-Ab'-Pd' ou une permutation cyclique de celui-ci, où Aa et Ab représentent des segments formant des superhélices qui forment des superhélices antiparallèles avec respectivement Aa' et Ab', et Pa à Pd représentent des segments formant des superhélices qui forment des superhélices parallèles avec Pa' à Pd', respectivement.

9. Polypeptide selon la revendication 7 ou 8, l'ordre séquentiel des segments formant des superhélices étant APH-P3-BCR-GCN-APH-P7-GCN-P4-P5-P8-BCR-P6 représenté par les SEQ ID N° 2, 8, 4, 6, 2, 16, 6, 10, 12, 18, 4 et 14, respectivement, ou une permutation cyclique de celui-ci.

10. Polypeptide selon la revendication 7 dans lequel trois des six côtés du tétraèdre sont composés de dimères de superhélices antiparallèles et de trois de dimères de superhélices parallèles.

11. Polypeptide de fusion d'un polypeptide formant un polyèdre selon l'une des revendications 1 à 10 avec un domaine polypeptidique fonctionnel fixé au segment N-et/ou C-terminal du polypeptide formant un polyèdre.

12. Polypeptide de fusion selon la revendication 11, dans lequel le domaine polypeptidique fonctionnel est divisé en deux segments qui sont liés à l'extrémité N-terminale et à l'extrémité C-terminale du polypeptide, respectivement, les deux segments du domaine de polypeptide fonctionnel se réassemblant lors de la formation d'un tétraèdre et d'une fonction de gain.

13. Polyèdre polypeptidique formé à partir d'un polypeptide selon l'une des revendications 1 à 10 ou d'un polypeptide de fusion selon l'une des revendications 11 à 12.

14. Polyèdre polypeptidique, dont les côtés sont composés de segments dimères formant des superhélices liés, le nombre desdits segments étant le double du nombre des côtés du polyèdre, le polypeptide formant un chemin fermé le long des côtés du polyèdre et des segments de liaison non hélicoïdaux étant situés entre les segments dimères formant des superhélices et formant les sommets du polyèdre.

15. ADN codant un polypeptide selon l'une des revendications 1 à 10, un polypeptide de fusion selon l'une des revendications 11 à 12, ou un polyèdre polypeptidique selon la revendication 13 ou 14.

16. Un médicament encagé dans une nanocage polyédrique formée à partir d'un polypeptide selon l'une des revendications 1 à 10 ou d'un polypeptide de fusion selon l'une des revendications 11 à 12 pour une utilisation en traitement ou pour le diagnostic d'un être humain ou d'un animal.

17. Le médicament encagé dans une nanocage polyédrique formée à partir d'un polypeptide selon l'une des revendications 1 à 10 ou d'un polypeptide de fusion selon l'une des revendications 11 à 12 pour l'utilisation selon la revendication 16, le polypeptide contenant des substances de liaison spécifiques à une cible dirigeant la nanocage polyédrique vers le tissu souhaité.
